# EUROPÄISCHE PATENTSCHRIFT

(11) **EP 2 604 203 B1**
(45) Veröffentlichungstag und Bekanntmachung des Hinweises auf die Patenterteilung: **09.11.2016**
(21) Anmeldenummer: 12195932.4
(22) Anmeldetag: 06.12.2012
(51) Int. Cl.: A61B 17/34, A61M 39/06, A61B 17/00

(54) **Dichteinrichtung zur Abdichtung einer Durchführung für ein medizinisches Instrument**
Sealing device for sealing a feedthrough for a medical instrument
Dispositif d'étanchéité pour étanchéifier un passage pour un instrument médical

(30) Priorität: 13.12.2011 DE 102011088337
(43) Veröffentlichungstag der Anmeldung: 19.06.2013
(73) Patentinhaber: Karl Storz GmbH & Co. KG, 78532 Tuttlingen (DE)
(72) Erfinder: Alonso, Jose Ramon, 80337 München (DE); Bacher, Uwe, 78532 Tuttlingen (DE); Oberländer, Martin, 78532 Tuttlingen (DE); Sauer, Michael, 78532 Tuttlingen (DE); Wagner, Sebastian, 78532 Tuttlingen (DE)

(56) Entgegenhaltungen:
- EP-A1- 1 671 598
- WO-A1-2006/118748
- WO-A1-2007/148959
- WO-A1-2011/060192
- WO-A2-01/54763
- DE-C1- 4 127 628
- DE-U1- 29 701 600
- FR-A1- 2 694 181
- US-A- 4 655 752
- US-A- 5 727 770
- US-B1- 6 379 372
- US-B1- 6 551 283

## Beschreibung

Die vorliegende Erfindung bezieht sich auf eine Dichteinrichtung zur Abdichtung einer Durchführung für ein medizinisches Instrument, insbesondere zur Abdichtung eines Trokars, durch den bei einem mikroinvasiven medizinischen Eingriff ein Endoskop und/oder andere medizinische Instrumente in eine natürliche oder künstliche Körperhöhle eingeführt werden können, oder eines Arbeitskanals eines Endoskops.

Ein Beispiel für mikroinvasive medizinische Methoden ist die Laparoskopie. Mittels eines Trokars wird ein künstlicher Zugang zur Bauchhöhle eines Patienten durch dessen Bauchdecke hindurch geschaffen. Durch das Lumen eines während des laparoskopischen Eingriffs in der Bauchdecke verbleibenden Tubus des Trokars können ein Endoskop und/oder weitere medizinische Instrumente (beispielsweise Zangen, Scheren, Nadelhalter) in die Bauchhöhle eingeführt werden. Während der Laparoskopie wird der Bauchraum mit Kohlenstoffdioxid oder einem anderen Gas gefüllt, um ein Pneumoperitoneum, einen den medizinischen Eingriff ermöglichenden Hohlraum zu schaffen. Ohne besondere Maßnahmen würde dieses Gas durch das Lumen des Tubus des Trokars entweichen. Deshalb wurden zahlreiche Ansätze entwickelt, um das Lumen des Tubus eines Trokars möglichst fluiddicht zu verschließen, und zwar sowohl im leeren Zustand als auch bei eingeführtem Instrument. Teilweise ähnliche Aufgabestellungen können sich an einem Arbeitskanal eines Endoskops oder beim Einführen eines Katheters in ein Blutgefäß ergeben. Im letztgenannten Fall ist jedoch nicht das Entweichen eines Gases, sondern von Blut zu verhindern.

In US 4,857,062 ist ein Ventil zum Einführen eines Katheters in eine Arterie beschrieben. Zur Abdichtung sind ein entenschnabelförmiges erstes Element und ein zweites flexibles Element, das zur Ausbildung einer fluiddichten Abdichtung mit einem Katheter komprimiert ist, vorgesehen. Beide Elemente sind hintereinander und starr in einem Gehäuse angeordnet.

In WO 93/01850 A1 ist eine durch Hebel betätigte Dichtung für einen Trokar beschrieben. Eine Wand aus einem Elastomer mit einer Öffnung wird beim Einführen eines Instruments in den Trokar durch mehrere Hebel gedehnt, wodurch die Öffnung vergrößert wird.

In US 5,366,446 ist eine Einführanordnung zur Verwendung auf der Haut eines Patienten beschrieben, die für das Einführen von Tuben mit unterschiedlichen Außendurchmessern ausgebildet ist. Die Anordnung umfasst eine Membran aus einem durchstechbaren elastomeren Material in der Mitte eines Faltenbalgs.

In EP 0 630 660 A1 ist eine Dichtanordnung zum Aufnehmen eines chirurgischen Instruments beschrieben. Die Dichtanordnung umfasst eine Entenschnabeldichtung oder eine Anordnung aus einer Mehrzahl von teilweise sternförmig geschlitzten Dichtelementen, die einander teilweise überlappen und teilweise konisch ausgebildet sind.

In EP 0 746 359 B1 ist ein Katheter-Sperrventil beschrieben. Zur Abdichtung sind eine Gummidichtung mit einer Öffnung und distal dieser ein Entenschnabelventil mit einem geraden Schlitz vorgesehen.

In WO 2010/045702 A1 ist eine Einwegdichtung für einen Trokar beschrieben. Die Einwegdichtung weist eine näherungsweise becherförmige Gestalt mit sich kreuzenden Schlitzen am Boden auf.

In US 4,430,081 ist eine Kanüle zur Verwendung mit Angiographiekathetern beschrieben. Zur Abdichtung gegen das Eindringen von Luft oder das Austreten von Blut aus einem Blutgefäß sind eine erste Dichtung mit einem Schlitz, eine zweite Dichtung mit einem Loch und eine dritte Dichtung mit einer Klappe darin vorgesehen, die aneinander angrenzend angeordnet sind.

In WO 91/12838 A1 ist ein Infusionszugang mit mehreren hintereinander angeordneten elastischen Scheiben, die jeweils kreisförmige Öffnungen oder gegeneinander verdreht angeordnete sternförmige Schlitze aufweisen, vorgesehen.

In EP 0 536 549 A1 ist eine Trokarhülse zum Durchführen eines medizinischen Instruments beschrieben. Eine Dichtungseinrichtung für eine Abdichtung eines Axialdurchgangs und eines Hohlschafts sowohl bei eingeführtem Instrument als auch bei nicht eingeführtem Instrument umfasst eine oder mehrere Trennwände aus elastischem Material mit gegeneinander versetzten Kreuzschlitzungen.

In WO 94/01149 A1 und DE 693 29 286 T2 ist ein Ventil für eine Einführungsanordnung beschrieben. Ein Körper aus Silikon oder einem anderen elastomeren Material umfasst eine zylindrische Wand, die eine Bohrung umschließt. Ein Ende der zylindrischen Wand und der Bohrung ist mit einer Wand mit einer zentral angeordneten Öffnung verschlossen. Das andere Ende der zylindrischen Wand und der Bohrung ist durch zwei gegeneinander geneigte Plättchen mit einem dazwischenliegenden Schlitz verschlossen.

In WO 98/32484 A1 ist ein Kathetereinführungsbesteck mit einem Hämostaseventil beschrieben. Ein Dichtelement umfasst zwei gelochte Stützscheiben, zwischen denen eine Dichtscheibe aus weich-elastischem Schaumkunststoff mit radial verlaufenden Schlitzen vorgesehen ist.

In EP 1 269 925 A1 ist eine Zugangskanüle für endoskopische Operationen beschrieben. Ein Doppelscheibenventil umfasst zwei Scheiben mit je einem dreistrahlig sternförmigen Schlitz, wobei die Schlitze der beiden Scheiben gegeneinander verdreht angeordnet sind.

In EP 1 350 476 A1 ist eine Trokarhülse mit einem Ventil beschrieben. Das Ventil umfasst einen Einführbereich mit in distaler Richtung aufeinander zulaufenden, als Schrägflächen ausgebildeten Wandabschnitten und elastisch aneinanderanliegenden Dichtlippen.

In DE 297 01 600 U1 ist ein Ventilmechanismus für medizinische Anwendungen beschrieben. Zwei gleichartige Ventilscheiben mit je einem zentral angeordneten Schlitz sind unter einem Winkel von 60 Grad bis 90 Grad in einem Gehäuse angeordnet.

In DE 601 05 973 T2 ist ein hämostatisches Ventil beschrieben, das eine proximale Ventildichtung mit einem Schlitz und eine distale Ventildichtung mit einem Schlitz umfasst. Ein Positioniervorsprung schränkt die relative Rotation beider Ventildichtungen ein.

In US 2006/0135977 A1 ist eine Trokar-Dichtstruktur mit zwei jeweils konischen Dichtabschnitten beschrieben.

In FR 2 694 181 A1 ist eine Vorrichtung zum automatischen Schließen einer Trokarhülse beschrieben. Zwei elastische Dichtscheiben mit je einem geraden Schlitz sind derart aneinander flächig angrenzend angeordnet, dass die beiden Schlitze orthogonal zueinander sind. Ferner ist proximal der beiden Dichtscheiben und an diese flächig angrenzend eine elastische Lochscheibe vorgesehen, deren Rand am äußeren Umfang eines in die Vorrichtung eingeführten Endoskops dichtend anliegen soll. Das Dokument offenbart eine Dichteinrichtung entsprechend dem Oberbegriff des Anspruchs 1.

In EP 1 671 598 A1 ist eine Dichtanordnung für einen Trokar beschrieben. Die Dichtanordnung umfasst zwei jeweils kegelförmige Dichtelemente mit je einem Schlitz, wobei die Schlitze nicht parallel zueinander angeordnet sind.

In WO 01/54763 A2 ist ein Hämostase-Ventil beschrieben, das zwei Dichtungen umfasst. Jede Dichtung weist einen Schlitz in einer relativ zur Längsachse der Vorrichtung geneigten Ebene auf. Formschluss zwischen den beiden Dichtungen gewährleistet eine orthogonale Ausrichtung der beiden Schlitze.

In US 6,551,283 B1 ist ein Hämostase-Ventil beschrieben, das zwei Elemente aus elastischem Material mit orthogonal zueinander angeordneten Schlitzen umfasst.

In WO 2007/148959 A1 ist eine Ventilanordnung für einen medizinischen Zugang beschrieben. Die Ventilanordnung umfasst ein erstes, kuppelförmig gewölbtes Ventil mit einem geraden Schlitz, das den Zugang verschließt, wenn kein medizinisches Instrument eingesetzt ist, und ein zweites Dichtventil mit einer zentralen Öffnung, das den Zugang verschließt, wenn ein medizinisches Instrument eingesetzt ist.

In WO 2011/060192 A1 ist eine Dichtanordnung mit einem kuppelförmigen oder halbkugelförmigen Dichtteil mit einem Schlitz zum Abdichten, wenn kein medizinisches Instrument eingesetzt ist, und einem kreisförmigen Dichtteil zum Dichten, wenn ein Instrument eingesetzt ist, beschrieben.

In US 6,379,372 B1 ist ein endovaskulares System mit einem scheibenförmigen Ventil und einem kuppelförmigen Ventil beschrieben.

In US 4,655,752 ist eine chirurgische Kanüle mit einem kreuzförmig geschlitzten konischen Dichtbauteil und einem weiteren konischen Dichtbauteil mit einer kreisförmigen Öffnung beschrieben.

In US 5,727,770 ist eine Doppelventil-Kanülendichtung mit einem geraden Schlitz in einem konischen Abschnitt zum Dichten, wenn keine Kanüle eingeführt ist, und einer kreisförmigen Öffnung in einem Membranventil zum Dichten, wenn eine Kanüle eingeführt ist, beschrieben.

In DE 41 27 628 C1 ist eine Einführschleuse für einen Katheder mit einem Y- bzw. sternförmigen Schlitz beschrieben.

In WO 2006/118748 A1 ist ein medizinisches Schlitzventil mit einem oder mehreren Schlitzen in einem abschnittsweise konischen und abschnittsweise pyramidalen Bauteil beschrieben.

Die beschriebenen Dichteinrichtungen weisen jeweils spezifische Vor- und Nachteile auf. Für viele Anwendungen sind noch keine befriedigenden Lösungen gefunden oder zumindest weitere Verbesserungen wünschenswert. Dies gilt insbesondere im Hinblick darauf, dass eine Dichteinrichtung zahlreiche Anforderungen gleichzeitig erfüllen soll. Dazu zählt, dass die Dichteinrichtung fluiddicht, robust, wiederverwendbar und dazu insbesondere autoklavierbar sein, einer Bewegung eines medizinischen Instruments in axialer Richtung möglichst geringen Widerstand entgegensetzen, eine Hebelmanipulation bzw. ein Kippen eines in die Dichteinrichtung eingesetzten medizinischen Instruments ermöglichen und auch dabei fluiddicht bleiben sowie kostengünstig herstellbar sein soll. Ferner sollen Dichteinrichtungen mit geringem Aufwand gereinigt werden können, einen kompakten Aufbau aufweisen, insbesondere eine geringe Baulänge (gemessen vom proximalen bis zum distalen Ende der Dichteinrichtung) und für medizinische Instrumente mit unterschiedlichen Querschnitten geeignet sein. Außerdem sollen medizinische Instrumente mit in Längsrichtung variierenden Querschnitten, insbesondere auch mit Hinterschnitten, Stufen, Absätzen und weiches Gewebe auch in Richtung von distal nach proximal die Dichteinrichtung passieren können, ohne zu verhaken oder auf andere Weise an der Dichteinrichtung hängen zu bleiben.

Eine Aufgabe der vorliegenden Erfindung besteht darin, eine verbesserte Dichteinrichtung zu schaffen, die insbesondere die aufgezählten Anforderungen und Erwartungen in ausgewogenen Verhältnissen erfüllt.

Diese Aufgabe wird durch den Gegenstand und die Merkmale des unabhängigen Anspruchs 1 gelöst.

Weiterbildungen sind in den abhängigen Ansprüchen angegeben.

Ausführungsformen der vorliegenden Erfindung beruhen auf der Idee, an einer Dichteinrichtung zwei aneinander anliegende Dichtmembranen mit je einem geraden oder zumindest unverzweigten Schlitz vorzusehen. Die Schlitze der beiden Dichtmembranen sind insbesondere in einem Winkel von ca. 90° zueinander angeordnet. Diese Ausgestaltung der Dichteinrichtung kann eine Dichtfunktion sowohl ohne medizinisches Instrument in der Dichteinrichtung als auch bei in die Dichteinrichtung eingeführtem medizinischen Instrument und für eine große Bandbreite von Durchmessern bzw. Querschnitten medizinischer Instrumente ermöglichen.

Eine Dichteinrichtung zur Abdichtung einer Durchführung für ein medizinisches Instrument umfasst eine erste Dichtmembran aus einem elastischen Material mit einem ersten Schlitz und eine zweite Dichtmembran aus einem elastischen Material mit einem zweiten Schlitz, wobei die erste Dichtmembran und die zweite Dichtmembran flächig aneinander anliegen, wobei die erste Dichtmembran und die zweite Dichtmembran so angeordnet sind, dass der erste Schlitz und der zweite Schlitz nicht parallel zueinander sind, wobei der erste Schlitz und der zweite Schlitz jeweils unverzweigt sind, und wobei die erste Dichtmembran und die zweite Dichtmembran jeweils zumindest in einem mittigen Bereich kuppelförmig gewölbt sind.

Die Dichteinrichtung ist insbesondere eine Dichteinrichtung für einen Trokar bzw. einen Tubus eines Trokars, der während eines mikroinvasiven Eingriffs einen Zugang zu einem Hohlraum bildet. Alternativ ist die Dichteinrichtung vorgesehen und ausgebildet für einen Arbeitskanal eines Endoskops oder für eine andere medizinische Vorrichtung, in die ein medizinisches Instrument eingeführt werden kann, und die mit und ohne das medizinische Instrument fluiddicht verschlossen sein soll.

Die Dichteinrichtung ist insbesondere zur lösbaren Befestigung am proximalen Ende eines Trokars oder eines Arbeitskanals eines Endoskops oder einer anderen medizinischen Vorrichtung vorgesehen und ausgebildet. Dazu umfasst die Dichteinrichtung insbesondere einen Befestigungsbereich mit einem elastischen Kragen oder einem anderen Oberflächenabschnitt, der eine formschlüssige mechanische Verbindung mit einem korrespondierend ausgebildeten Oberflächenabschnitt an der medizinischen Vorrichtung ermöglicht.

Die erste Dichtmembran und die zweite Dichtmembran sind insbesondere im Wesentlichen aus Silikon oder einem ähnlichen elastischen, biokompatiblen und autoklavierbaren Material gebildet. Der erste Schlitz und der zweite Schlitz sind jeweils durch zwei Dichtlippen der entsprechenden Dichtmembran gebildet oder begrenzt, wobei die beiden Dichtlippen jeweils im entspannten Zustand der Dichtmembran aneinander anliegen oder allenfalls einen sehr geringen Abstand aufweisen. Die erste Dichtmembran und die zweite Dichtmembran liegen insbesondere vollflächig oder nahezu vollflächig aneinander an. Die Schlitze in den Dichtmembranen sind jeweils insofern unverzweigt, als sie insbesondere nicht kreuzförmig oder sternförmig ausgebildet sind.

Die Dichteinrichtung kann eine dritte Dichtmembran aufweisen. Die dritte Dichtmembran liegt insbesondere unmittelbar und vollflächig an der ersten Dichtmembran oder an der zweiten Dichtmembran an. Ferner kann die Dichteinrichtung eine vierte oder weitere Dichtmembranen aufweisen, die zusammen mit der ersten Dichtmembran, der zweiten Dichtmembran und der dritten Dichtmembran einen Stapel bilden können. Insbesondere liegt jede Dichtmembran an der oder den jeweils unmittelbar benachbarten Dichtmembranen vollflächig an.

Die einen unverzweigten Schlitz bildenden, im entspannten Zustand geraden oder leicht gekrümmten Dichtlippen können an zwei gegenüberliegenden Bereichen eines Umfangs eines medizinischen Instruments, in der Regel jedoch nicht am gesamten Umfang des medizinischen Instruments anliegen. Dichtlippen, die zwei gegeneinander verdreht angeordnete Schlitze in zwei Dichtmembranen bilden, können bei hinreichend elastischer Ausgestaltung zusammengenommen am gesamten Umfang eines medizinischen Instruments anliegen. Wenn die beiden Dichtmembranen aneinander anliegen, können die vier Dichtlippen, die die beiden Schlitze in den beiden Dichtmembranen bilden, zusammengenommen am gesamten Umfang eines medizinischen Instruments anliegen und so eine weitgehend oder vollständig fluiddichte Anordnung bilden.

Herkömmliche Dichteinrichtungen mit sternförmigen Schlitzen leisten dies in der Regel in geringerem Maße. Intuitiv mögen sternförmige Schlitze naheliegend sein (und sind im eingangs zusammengefassten Stand der Technik auch zahlreich beschrieben), da die zungenförmigen Bereiche der Dichtmembran zwischen den radialen Abschnitten des sternförmigen Schlitzes beim Einführen eines medizinischen Instruments in erster Linie elastisch gebogen und nur in geringerem Maße gedehnt oder geschert werden. Tatsächlich kann die Verwendung je eines unverzweigten Schlitzes in jeder Dichtmembran eine höhere Elastizität hinsichtlich Dehnung und Scherung voraussetzen, gleichzeitig jedoch auch einen Grad der Fluiddichtheit ermöglichen, der bei sternförmigen Schlitzen nicht erreichbar ist.

Auch bei einer Umkehrung einer Bewegungsrichtung eines medizinischen Instruments in der Dichteinrichtung (Einführen/Herausziehen) können zwei jeweils unverzweigte und gegeneinander verdreht angeordnete Schlitze in zwei aneinander anliegenden Dichtmembranen vorteilhaft sein. Insbesondere kann, abhängig von der Reibung zwischen den Dichtlippen und dem medizinischen Instrument, ein Umklappen der Dichtlippen besonders weich und damit hinsichtlich der Dichtfunktion vorteilhaft ablaufen oder ganz entfallen.

Ferner kann die Dichteinrichtung einen radialen Ausgleich für kleine Querschnitte einzuführender medizinischer Instrumente ermöglichen und für einen großen Durchmesserbereich bzw. zahlreiche verschiedene Querschnitte medizinischer Instrumente geeignet sein. Medizinische Instrumente, die am distalen Ende einen größeren Querschnitt als proximal des distalen Endes und/oder Hinterschneidungen aufweisen, und Gewebe können einfacher und mit einem geringeren Risiko einer Komplikation aus der Dichteinrichtung herausgezogen werden. Anders als viele herkömmliche Dichteinrichtungen weisen die hier beschriebenen Dichteinrichtungen im geringeren Maße oder gar nicht die Eigenschaft einer "Mausefalle" für medizinische Instrumente und andere Gegenstände mit nicht vollständig zylindrischer Gestalt auf.

Eine hier beschriebene Dichteinrichtung kann gleichzeitig die Funktion eines fluiddichten Verschlusses für eine medizinische Vorrichtung, wenn kein medizinisches Instrument eingeführt ist, und die Funktion einer fluiddichten Abdichtung, wenn ein medizinisches Instrument eingeführt ist, bieten. Dabei kann die Dichteinrichtung eine verhältnismäßig geringe Baulänge und einen einfachen Aufbau aus wenigen Bauteilen aufweisen. Insbesondere umfasst die Dichteinrichtung nur zwei Bauteile.

Bei einer Dichteinrichtung, wie sie hier beschrieben ist, nimmt insbesondere zumindest entweder die Dicke der ersten Dichtmembran zum ersten Schlitz hin oder die Dicke der zweiten Dichtmembran zum zweiten Schlitz hin kontinuierlich oder diskontinuierlich ab.

Bei einer Dichteinrichtung, wie sie hier beschrieben ist, nimmt insbesondere zumindest entweder die Dicke der ersten Dichtmembran in beiderseits an den ersten Schlitz angrenzenden Bereichen keilförmig zum ersten Schlitz hin oder die Dicke der zweiten Dichtmembran in beiderseits an den zweiten Schlitz angrenzenden Bereichen keilförmig zum zweiten Schlitz hin ab.

Die an die Schlitze angrenzenden Bereiche, in denen die Dicke der ersten Dichtmembran bzw. die Dicke der zweiten Dichtmembran keilförmig abnimmt, sind insbesondere jeweils streifenförmig oder rechteckig. Die minimale Dicke der ersten Dichtmembran bzw. der zweiten Dichtmembran unmittelbar am entsprechenden Schlitz bzw. an der Kante der entsprechenden Dichtlippe beträgt insbesondere 0,2 mm oder weniger. Der Winkel zwischen den sich zum Schlitz hin aneinander annähernden proximalen und distalen Oberflächen der Dichtmembran beträgt insbesondere 30° oder weniger.

Durch ein dünnes Auslaufen der Dichtmembrane zu den entsprechenden Schlitzen hin bzw. durch die beschriebene diskontinuierliche, kontinuierliche oder keilförmige Abnahme der Dicken der Dichtmembranen ist die Elastizität der durch die Dichtmembranen gebildeten Dichtlippen in weiten Bereichen einstellbar. Durch die beschriebene Ausgestaltung der Dichtlippen und die Wahl des Materials, insbesondere seiner Elastizität und Härte, kann erreicht werden, dass für medizinische Instrumente mit Querschnitten und Durchmessern innerhalb eines vergleichsweise großen Bereichs jede der Dichtlippen jeweils über einen Bereich von mehr als 90° am Umfang des medizinischen Instruments anliegt. Bei geeigneter relativer Anordnung der Schlitze in den beiden Dichtmembranen, und da die beiden Dichtmembranen ausgebildet sind, um flächig aneinander anzuliegen, kann die Dichtmembranen zusammengenommen lückenlos oder im Wesentlichen lückenlos am gesamten Umfang des medizinischen Instruments anliegen.

Die diskontinuierliche oder kontinuierliche und insbesondere keilförmige Abnahme der Dicken der Dichtmembranen zu den Schlitzen hin und die resultierende Zunahme der Elastizität der Dichtmembranen zu den Schlitzen hin können ferner das Risiko einer Zerstörung oder Beschädigung der Dichtmembranen, insbesondere durch spitze oder scharfkantige medizinische Instrumente und andere Gegenstände, verringern.

Zur weiteren Verbesserung der Robustheit bzw. zur Minderung des Risikos einer Zerstörung oder Beschädigung kann die Dichteinrichtung in der Umgebung der Schlitze durch eine Beschichtung oder durch Einrichtungen aus einem zäheren oder härteren Material (beispielsweise Polymer, Metall oder Keramik) verstärkt sein.

Bei einer Dichteinrichtung, wie sie hier beschrieben ist, sind insbesondere die Länge des ersten Schlitzes und die Länge des zweiten Schlitzes jeweils mindestens doppelt so groß wie der größte Durchmesser eines Abschnitts eines medizinischen Instruments, für den die Dichteinrichtung vorgesehen ist.

Der größte Durchmesser eines Abschnitts eines medizinischen Instruments, für den die Dichteinrichtung vorgesehen ist, ergibt sich insbesondere aus dem Querschnitt bzw. dem Durchmesser des Lumens eines Tubus eines Trokars, für den die Dichteinrichtung vorgesehen und ausgebildet ist. Wenn die Längen der Schlitze mindestens doppelt so groß wie der größte Durchmesser des genannten Abschnitts eines medizinischen Instruments ist, kann die beschriebene Dichtwirkung insbesondere das Anliegen jeder einzelnen Dichtlippe über einen Bereich von mindestens 90°, besonders gut erreichbar sein.

Bei einer Dichteinrichtung, wie sie hier beschrieben ist, sind die erste Dichtmembran und die zweite Dichtmembran jeweils insbesondere so ausgebildet, dass jede an einen Schlitz angrenzende Dichtlippe am größten kreisförmigen Querschnitt eines medizinischen Instruments, für den die Dichteinrichtung vorgesehen ist, jeweils in einem Bereich anliegt, der sich in umfänglicher Richtung über mindestens 90° erstreckt.

Wie bereits erwähnt, ermöglicht eine derartige Ausgestaltung der Dichtmembranen, dass die Dichtlippen zusammen lückenlos oder im Wesentlichen lückenlos am gesamten Umfang eines medizinischen Instruments anliegen und auf diese Weise fluiddicht abdichtend wirken.

Bei einer Dichteinrichtung, wie sie hier beschrieben ist, umfasst insbesondere eine formschlüssige Verbindung der ersten Dichtmembran und der zweiten Dichtmembran.

Bei einer Dichteinrichtung, wie sie hier beschrieben ist, sind die erste Dichtmembran und die zweite Dichtmembran insbesondere mittels einer oder mehrerer Rastverbindungen miteinander mechanisch verbunden.

Insbesondere sind die erste Dichtmembran und die zweite Dichtmembran mittels zweier Rastverbindungen, die einander gegenüberliegend angeordnet sind, miteinander mechanisch verbunden. Alternativ sind die erste Dichtmembran und die zweite Dichtmembran mittels dreier oder vierer Rastverbindungen, die voneinander gleiche Abstände aufweisen, miteinander mechanisch verbunden.

Jede Rastverbindung umfasst insbesondere eine Rastnase, die mit einer der beiden Dichtmembranen dauerhaft verbunden ist und beispielsweise in eine Ausnehmung oder eine Öse an der anderen Dichtmembran eingreifen kann. Die Rastnase und die Ausnehmung, die Öse oder eine andere zur Rastnase korrespondierende Einrichtung sind jeweils insbesondere an einem Rahmen angeordnet, der mit jeweils einer der beiden Dichtmembranen verbunden ist. Der Rahmen kann ein anderes Material aufweisen als die zugeordnete Dichtmembran und mit dieser geklebt, geschweißt oder auf andere Weise stoffschlüssig gefügt sein. Insbesondere weist der Rahmen ein Material auf, das steifer oder weniger elastisch ist als das Material der zugeordneten Dichtmembran. Alternativ weist der Rahmen das gleiche Material auf wie die zugeordnete Dichtmembran. In diesem Fall bewirkt insbesondere eine größere Materialstärke im Bereich des Rahmens eine geringere Elastizität des Rahmens. Bei einer Dichteinrichtung, wie sie hier beschrieben ist, sind die erste Dichtmembran und die zweite Dichtmembran insbesondere so angeordnet, dass der erste Schlitz und der zweite Schlitz in einem Winkel von mindestens 60° oder von mindestens 80° oder in einem Winkel von 90° zueinander angeordnet sind.

Bei einer Dichteinrichtung, wie sie hier beschrieben ist, sind insbesondere der erste Schlitz und der zweite Schlitz jeweils gerade oder bogenförmig mit einem Krümmungsradius, der mindestens so groß ist, wie eine Länge, eine Breite oder ein Durchmesser der ersten Dichtmembran oder der zweiten Dichtmembran.

Im Fall gewölbter Dichtmembranen wird ein Schlitz als gerade bezeichnet, wenn er in einer Ebene liegt, die in der Mitte des Schlitzes oder an allen Orten des Schlitzes senkrecht zur Dichtmembran ist. Eine gerade oder nur leicht gekrümmte Ausgestaltung der Schlitze kann die Dichtwirkung der Dichteinrichtung fördern.

Insbesondere sind die erste Dichtmembran und die zweite Dichtmembran jeweils zumindest in einem kreisförmigen Bereich, dessen Durchmesser der Länge des ersten Schlitzes oder der Länge des zweiten Schlitzes entspricht, kuppelförmig gewölbt. Insbesondere sind die erste Dichtmembran und die zweite Dichtmembran jeweils im Wesentlichen vollständig kuppelförmig gewölbt.

Bei einer Dichteinrichtung, wie sie hier beschrieben ist, weisen die erste Dichtmembran und die zweite Dichtmembran insbesondere jeweils einen an die Schlitze angrenzenden kugelflächenförmigen Oberflächenabschnitt auf.

Der mittige Bereich einer Membran ist kuppelförmig gewölbt, wenn die gaußsche Krümmung durchgehend positiv ist. Der kuppelförmig gewölbte Bereich bzw. der kugelflächenförmige Oberflächenabschnitt weist jeweils insbesondere einen kreisförmigen Rand auf. Eine kuppelförmige und insbesondere kugelflächenförmige Ausgestaltung der Dichtmembranen in der Umgebung der Schlitze kann die beschriebene Dichtwirkung unterstützen. Ferner können die Dichtmembranen außerhalb des kuppelförmig gewölbten Bereichs bzw. außerhalb der Bereiche, innerhalb derer sie kugelflächenförmige Oberflächenabschnitte aufweisen, trichterförmig ausgeführt sein. Die Kuppelform und insbesondere die Kugelflächenform sowie gegebenenfalls eine trichterförmige bzw. konische Ausgestaltung können das Einführen eines medizinischen Instruments in die Dichteinrichtung vereinfachen. Ferner kann das Risiko einer Beschädigung durch ein spitzes oder scharfkantiges medizinisches Instrument verhindert werden, indem die Spitze des medizinischen Instruments an der schrägen Oberfläche der proximalen Dichtmembran entlang gleitend zu den Schlitzen geführt wird.

Bei einer Dichteinrichtung, wie sie hier beschrieben ist, sind insbesondere am äußeren Rand der ersten Dichtmembran ein erstes Randprofil und am äußeren Rand der zweiten Dichtmembran ein zweites Randprofil vorgesehen, wobei das erste Randprofil und das zweite Randprofil für eine formschlüssige Verbindung der ersten Dichtmembran und der zweiten Dichtmembran ausgebildet sind.

Insbesondere sind eines der beiden Randprofile als nach radial außen vorstehender Kragen und das andere der beiden Randprofile als nach radial innen offene Nut ausgebildet, wobei der Kragen und die Nut korrespondierende Querschnitte aufweisen. Derartige und ähnliche Randprofile ermöglichen eine formschlüssige mechanische Verbindung der Dichtmembranen, die durch elastische Verformung der beiden Dichtmembranen herstellbar und lösbar sein kann. Auf diese Weise kann die Dichteinrichtung mit geringem Aufwand zur Reinigung und Sterilisierung zerlegt und danach wieder zusammengesetzt werden. Werkzeug ist nicht erforderlich. Um die Zerlegung der Dichteinrichtung weiter zu vereinfachen, kann am äußeren Rand von einer der beiden Dichtmembranen eine Lasche oder eine andere Einrichtung vorgesehen sein, die eine Trennung der Randprofile vereinfacht. Eine Zerlegbarkeit kann die Wiederverwendbarkeit der Dichteinrichtung ermöglichen oder fördern.

Bei einer Dichteinrichtung, wie sie hier beschrieben ist, weichen insbesondere sowohl der äußere Rand der ersten Dichtmembran als auch der äußere Rand der zweiten Dichtmembran jeweils an zumindest einer Stelle von einer Kreissymmetrie so ab, dass eine vorbestimmte relative Orientierung der Dichtmembranen vorgegeben ist.

Beispielsweise sind an der ersten Dichtmembran eine Nase und an der zweiten Dichtmembran eine korrespondierende Ausnehmung vorgesehen, so dass die erste Dichtmembran und die zweite Dichtmembran nur derart miteinander verbunden werden können, dass die Nase in der Ausnehmung angeordnet wird. Durch diese oder ähnliche Merkmale kann erreicht werden, dass die Schlitze in den Dichtmembranen immer die vorgesehene relative Orientierung aufweisen, insbesondere im rechten Winkel zueinander angeordnet sind.

Eine Dichteinrichtung, wie sie hier beschrieben ist, weist insbesondere ferner zumindest entweder eine Verstärkungsrippe an der ersten Dichtmembran oder eine Verstärkungsrippe an der zweiten Dichtmembran auf.

Eine Verstärkungsrippe ist insbesondere im Wesentlichen senkrecht (mit einem Winkel von mindestens 60 Grad) und insbesondere im Wesentlichen mittig zum ersten Schlitz bzw. zum zweiten Schlitz angeordnet. Mit Verstärkungsrippen an der ersten und/oder zweiten Dichtmembran können die elastischen Verformbarkeiten der Dichtmembranen vorteilhaft beeinflusst bzw. eingestellt werden. Die Verstärkungsrippen können jeweils einen Querschnitt in Gestalt eines Rechtecks, eines Halbkreises, eines Teils einer Ellipse oder in anderer Gestalt haben. Der Querschnitt einer Verstärkungsrippe kann jeweils entlang der Verstärkungsrippe variieren. Insbesondere ist an einer Dichtmembran beiderseits des Schlitzes je eine Verstärkungsrippe symmetrisch zueinander angeordnet. Alternativ können beiderseits des Schlitzes jeweils mehrere Verstärkungsrippen angeordnet sein.

Bei einer Dichteinrichtung, wie sie hier beschrieben ist, ist insbesondere zumindest entweder die erste Dichtmembran oder die zweite Dichtmembran zumindest an einer Seite reibungsmindernd beschichtet.

Eine reibungsmindernde Beschichtung der Dichteinrichtung zumindest in der Nähe der Schlitze kann die zum Bewegen eines medizinischen Instruments in der Dichteinrichtung zur Überwindung der Haft- und der Gleitreibung erforderlichen Kräfte herabsetzen und darüber hinaus bei einer Umkehr der Bewegungsrichtung ein Umklappen der Dichtlippen verhindern. Eine durch die Beschichtung verminderte Reibung kann ferner das Risiko einer Beschädigung oder Zerstörung der Dichteinrichtung, beispielsweise beim Einführen eines spitzen oder scharfkantigen medizinischen Instruments, vermindern. Eine Beschichtung der aneinander anliegenden gegenüberliegenden Oberflächen der Dichtmembranen kann ferner das Anliegen der Dichtlippen an ein in die Dichteinrichtung eingeführtes medizinisches Instrument und damit die Dichtwirkung fördern. Dabei sind insbesondere jeweils beide (proximale und distale) Oberflächen beider Dichtmembranen oder alle Oberflächen der Dichteinrichtung beschichtet.

Die reibungsmindernde Beschichtung der Dichteinrichtung umfasst insbesondere Poly(p-Xylylene), das auch unter dem Markennamen Parylene vertrieben wird.

Eine Dichteinrichtung, wie sie hier beschrieben ist, umfasst insbesondere ferner einen Befestigungsbereich zur Befestigung der Dichteinrichtung an einem Trokar oder einer anderen medizinischen Vorrichtung und einen die zweite Dichtmembran umgebenden elastischen mantelförmigen Wandabschnitt, der die zweite Dichtmembran mit dem Befestigungsbereich verbindet, und dessen Elastizität eine Bewegung der Dichtmembran relativ zum Befestigungsbereich ermöglicht.

Der elastische mantelförmige Wandabschnitt weist insbesondere im Wesentlichen die Gestalt eines ringförmigen Ausschnitts eines Kreiszylindermantels oder eines Kegelmantels auf. Der elastische mantelförmige Wandabschnitt kann eine konstante Wanddicke oder eine von proximal nach distal zunehmende oder abnehmende Wanddicke aufweisen. Ein erster Rand des elastischen mantelförmigen Wandabschnitts ist insbesondere mit dem äußeren Rand der zweiten Dichtmembran verbunden. Ein zweiter Rand des elastischen mantelförmigen Wandabschnitts ist mit dem Befestigungsbereich verbunden. Der erste Rand und der zweite Rand des elastischen mantelförmigen Wandabschnitts sind insbesondere jeweils kreisförmig. Der elastische mantelförmige Wandabschnitt und die zweite Dichtmembran und/oder der Befestigungsbereich sind insbesondere einstückig ausgebildet. Der elastische mantelförmige Wandabschnitt weist insbesondere die Funktion eines Faltenbalgs auf oder ist Teil eines Faltenbalgs.

Die Elastizität des elastischen mantelförmigen Wandabschnitts kann eine Bewegung der Dichtmembranen relativ zum Befestigungsbereich ermöglichen, insbesondere eine Verschiebung der Dichtmembranen in Richtungen senkrecht zur Einführrichtung und/oder ein Kippen oder Schwenken der Dichtmembranen relativ zum Befestigungsbereich. Dadurch kann die Elastizität des elastischen mantelförmigen Wandabschnitts bei Kipp- oder Hebelbewegungen des medizinischen Instruments in der Dichteinrichtung die resultierenden Kräfte zwischen dem medizinischen Instrument und der Dichteinrichtung, insbesondere den Dichtlippen, reduzieren. Dies fördert die Dichtwirkung der Dichteinrichtung und mindert das Risiko einer Beschädigung der Dichteinrichtung. Eine wiederverwendbare Ausgestaltung der Dichteinrichtung kann deshalb sinnvoll sein und zu niedrigen Lebenszykluskosten beitragen.

Ein Trokar umfasst eine Dichteinrichtung, wie sie hier beschrieben ist.

Der Trokar weist insbesondere außer der Dichteinrichtung keine weitere Dichteinrichtung zum Verschluss des Trokars bei in den Trokar eingesetzten medizinischem Instrument oder zum Verschluss des Trokars ohne eingesetztes medizinisches Instrument auf.

### Kurzbeschreibung der Figuren

Nachfolgend werden Beispiele und Ausführungsformen anhand der beigefügten Figuren näher erläutert.

Es zeigen:
- Figur 1: eine schematische Darstellung eines Trokars mit einem medizinischen Instrument;
- Figur 2: eine weitere schematische Darstellung des Trokars und des medizinischen Instruments aus Figur 1;
- Figur 3: eine schematische axonometrische Darstellung einer beispielhaften Dichteinrichtung, welche nicht Teil der vorliegenden beanspruchten Erfindung ist;
- Figur 4: eine weitere schematische axonometrische Darstellung der beispielhaften Dichteinrichtung aus Figur 3;
- Figur 5: eine weitere schematische Darstellung einer beispielhaften Dichtmembran aus den Figuren 3 und 4;
- Figur 6: eine schematische Darstellung der beispielhaften Dichtmembranen aus den Figuren 3 und 4;
- Figur 7: eine schematische Darstellung einer weiteren beispielhaften Dichtmembran;
- Figur 8: eine schematische Darstellung einer erfindungsgemäßen Dichteinrichtung;
- Figur 9: eine weitere schematische Darstellung eines ersten erfindungsgemäßen Dichtmembranbauteils aus Figur 8;
- Figur 10: eine weitere schematische Darstellung eines zweiten erfindungsgemäßen Dichtmembranbauteils aus Figur 8;
- Figur 11: eine schematische axonometrische Darstellung einer weiteren erfindungsgemäßen Dichteinrichtung an einem Ende eines Trokars;
- Figur 12: eine weitere schematische axonometrische Darstellung der Dichteinrichtung aus Figur 11;
- Figur 13: eine schematische Schnittdarstellung einer weiteren erfindungsgemäßen Dichteinrichtung;
- Figur 14: eine schematische axonometrische Schnittdarstellung eines weiteren Trokars mit einer weiteren erfindungsgemäßen Dichteinrichtung;
- Figur 15: eine schematische Schnittdarstellung der erfindungsgemäßen Dichteinrichtung aus Figur 14;
- Figur 16: eine weitere schematische axonometrische Schnittdarstellung der Dichteinrichtung aus den Figuren 14 und 15;
- Figur 17: eine weitere schematische axonometrische Schnittdarstellung der Dichteinrichtung aus den Figuren 14 bis 16.

Beschreibung von beispielhaften Dichtmembranen, welche nicht Teil der beanspruchten Erfindung sind, jedoch zu deren Verständnis beitragen.

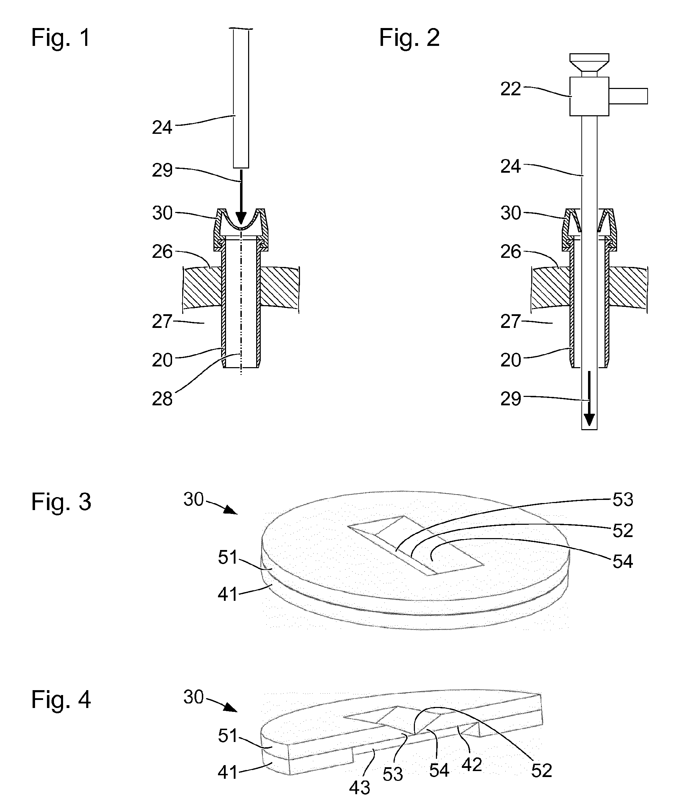

Die Figuren 1 und 2 zeigen schematische Schnittdarstellungen eines Trokars 20 bzw. des Tubus eines Trokars, der in eine Bauchdecke 26 eines Patienten eingesetzt ist. Durch das Lumen des Trokars 20 hindurch können ein oder mehrere medizinische Instrumente in einen Hohlraum 27 unter der Bauchdecke 26 des Patienten eingeführt werden. Der Trokar 20, die Bauchdecke 26 und der Hohlraum 27 unter der Bauchdecke 26 sind in einem Schnitt entlang einer Ebene, die eine Längsachse 28 des Trokars 20 enthält, dargestellt. Der Trokar 20 ist insbesondere zumindest teilweise rotationssymmetrisch zur Längsachse 28. Der Trokar 20 umfasst eine Dichteinrichtung 30 an seinem proximalen Ende außerhalb des Hohlraums 27. Beispiele der Dichteinrichtung 30 sind mit Bezug auf die
Figuren 3 bis 8 näher beschrieben.

Ferner ist in den Figuren 1 und 2 ein medizinisches Instrument 22 mit einem Schaft 24 dargestellt. Da der innere Aufbau des medizinischen Instruments 22 und von dessen Schaft 24 für die nachfolgend beschriebenen Eigenschaften des Trokars 20 unerheblich sind, sind der Einfachheit halber nur Konturen des medizinischen Instruments 22 und von dessen Schaft 24 dargestellt. Das medizinische Instrument 22 ist in Figur 2 beispielhaft als Endoskop dargestellt.

Der Schaft 24 des medizinischen Instruments 22 kann in einer Richtung 29 parallel oder im Wesentlichen parallel zur Längsachse 28 des Trokars 20 in den Trokar 20 eingeführt werden. Der Schaft 24 des medizinischen Instruments 22 ist in Figur 1 vor dem Einführen in den Trokar 20 bzw. in dessen Lumen und in Figur 2 nach dem Einführen in den Trokar 20 bzw. dessen Lumen dargestellt. Die Dichteinrichtung 30 verschließt das proximale Ende des Trokars 20 sowohl dann, wenn, wie in Figur 1 dargestellt, kein medizinisches Instrument in den Trokar 20 eingeführt ist, als auch dann, wenn, wie in Figur 2 dargestellt, ein medizinisches Instrument 22 in den Trokar 20 eingeführt ist, fluiddicht. Dadurch verhindert die Dichteinrichtung 30 einen Austritt eines Gases oder eines anderen Fluids aus dem Hohlraum 27 unter der Bauchdecke 26 des Patienten.

Figur 3 zeigt eine schematische axonometrische Darstellung eines einfachen Beispiels einer Dichteinrichtung 30 mit einer ersten Dichtmembran 41 und einer zweiten Dichtmembran 51. Die erste Dichtmembran 41 und die zweite Dichtemembran 51 sind bei dem dargestellten Beispiel jeweils im Wesentlichen eben, plattenförmig und kreisförmig. Beide Dichtmembranen 41,51 grenzen unmittelbar aneinander an, d.h. eine im Wesentlichen ebene Oberfläche der ersten Dichtmembran 41 liegt vollflächig an einer im Wesentlichen ebenen Oberfläche der zweiten Dichtmembran 51 an. In der Mitte der zweiten Dichtmembran 51 ist ein gerader Schlitz 52 zwischen zwei Dichtlippen 53, 54 angeordnet. In der Mitte der ersten Dichtmembran 41 ist ein weiterer Schlitz zwischen zwei Dichtlippen angeordnet, der in Figur 3 nicht sichtbar ist.

Figur 4 zeigt eine weitere schematische axonometrische Darstellung der Dichteinrichtung 30 aus Figur 3. Im Gegensatz zu Figur 3 ist die Dichteinrichtung 30 in Figur 4 entlang einer Ebene, die den Schlitz 42 in der ersten Dichtmembran 41 enthält, aufgeschnitten dargestellt. Der Schlitz 52 in der zweiten Dichtmembran 51 ist senkrecht zum Schlitz 42 in der ersten Dichtmembran 41 und damit auch senkrecht zur Schnittebene.

In dem dargestellten Schnitt ist erkennbar, dass die Dichtlippen 53, 54 der zweiten Dichtmembran 51 durch Bereiche der zweiten Dichtmembran 51 gebildet werden, die sich keilförmig zum Schlitz 52 hin verjüngen. Anders ausgedrückt ist die Dicke der zweiten Dichtmembran 51 in an den Schlitz 52 angrenzenden streifenförmigen bzw. rechteckigen Bereichen jeweils eine lineare oder affin-lineare Funktion des Abstands vom Schlitz 52. Entsprechendes gilt für eine Dichtlippe 43 und eine weitere, in Figur 4 nicht dargestellte Dichtlippe der ersten Dichtmembran 41, die den Schlitz in der ersten Dichtmembran 41 bilden. Dabei sind die an die erste Dichtmembran 41 angrenzende Oberfläche der zweiten Dichtmembran 51 und die an die zweite Dichtmembran 51 angrenzende Oberfläche der ersten Dichtmembran 41 jeweils eben, so dass die Dichtmembranen 41,51 einander vollflächig berühren.

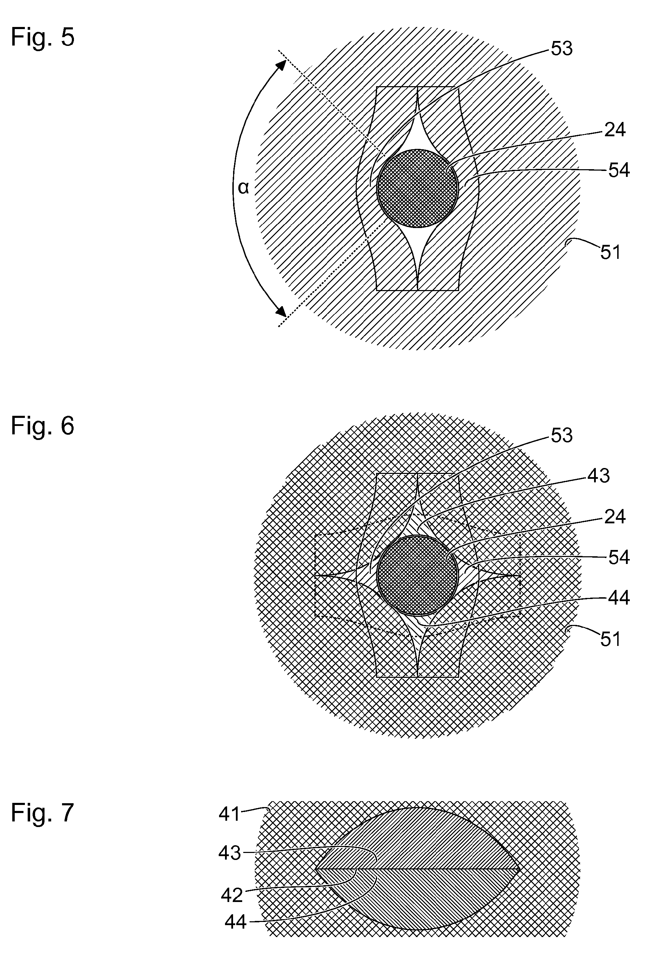

Figur 5 zeigt eine weitere schematische Darstellung der zweiten Dichtmembran 51. Die Zeichenebene der Figur 5 ist senkrecht zur Längsachse 28 und zur Einführrichtung 29 eines Tubus eines Trokars, für den die Dichteinrichtung vorgesehen ist (vgl. Figuren 1 und 2) und parallel zu der vom Betrachter abgewandten ebenen Oberfläche der zweiten Dichtmembran 51, die dazu ausgebildet ist, an der in Figur 5 nicht dargestellten ersten Dichtmembran anzuliegen.

Figur 5 zeigt die zweite Dichtmembran 51 zusammen mit einem im Querschnitt dargestellten Schaft 24 eines medizinischen Instruments. Die Schraffur der zweiten Dichtmembran 51 deutet in Figur 5 (wie auch in Figur 6) keine Schnittfläche an, sondern dient der besseren Erkennbarkeit der ersten Dichtmembran 51. Der Schaft 24 ist in den Schlitz zwischen den Dichtlippen 53, 54 der zweiten Dichtmembran 51 eingeführt. Durch den Schaft 24 sind die zweite Dichtmembran 51 und insbesondere deren Dichtlippen 53, 54 elastisch verformt.

Jede der Dichtlippen 53, 54 liegt in einem Bereich am Umfang des Schafts 24 des medizinischen Instruments an, der sich in umfänglicher Richtung über einen Winkel α (alpha) erstreckt. Die zweite Dichtmembran 51, insbesondere das Material, die Dicke bzw. Materialstärke, die Breite und Länge der die Dichtlippen 53, 54 bildenden, im entspannten Zustand rechteckigen Bereiche der zweiten Dichtmembran 51 und die Keilform (Öffnungswinkel bzw. Ortsabhängigkeit der Dicke) der die Dichtlippen 53, 54 bildenden Bereiche sind so ausgebildet, dass der Winkel α (alpha) mindestens 90° beträgt. Entsprechendes gilt für die in Figur 5 nicht dargestellte erste Dichtmembran 41.

Figur 6 zeigt eine hinsichtlich der Zeichenebene und der dargestellten Blickrichtung der Figur 5 entsprechende schematische Darstellung beider Dichtmembranen 41, 51. Die erste Dichtmembran 41 ist dabei an der vom Betrachter abgewandten Seite der zweiten Dichtmembran 51 angeordnet und deshalb von dieser weitgehend verdeckt. Die beiden Dichtmembranen 41, 51 sind jedoch anhand ihrer Schraffur unterscheidbar. Die erste Dichtmembran 41 ist von links oben nach rechts unten schraffiert, die zweite Dichtmembran 51 ist von links unten nach rechts oben schraffiert. Wo beide Dichtmembranen 41,51 einander überdecken bzw. überlappen, entsteht eine enge Kreuzschraffur.

Es ist erkennbar, dass am gesamten Umfang des Schafts 24 des medizinischen Instruments abwechselnd und lückenlos die Dichtlippen 43, 44, 53, 54 der beiden Dichtmembranen 41, 51 anliegen. Dies wird dadurch bedingt, dass einerseits, wie bereits oben anhand der Figur 5 dargestellt, jede Dichtlippe 53, 54 der zweiten Dichtmembran 51 und in gleicher Weise jede Dichtlippe 43, 44 der ersten Dichtmembran 41 in einem Bereich am Schaft 24 des medizinischen Instruments anliegt, der in umfänglicher Richtung sich über mindestens 90° erstreckt, und dass andererseits die erste Dichtmembran 41 und die zweite Dichtmembran 51 und die durch die Dichtlippen 43, 44, 53, 54 gebildeten Schlitze in denselben um 90° gegeneinander verdreht angeordnet sind.

Figur 7 zeigt eine schematische Darstellung eines weiteren Beispiels einer Dichtmembran 41, die in einigen Merkmalen den oben anhand der Figuren 3 bis 6 dargestellten Dichtmembranen ähnelt. Die Darstellung in Figur 7 ähnelt hinsichtlich der Zeichenebene den Figuren 5 und 6, die Schraffuren sind jedoch anders gewählt. In Kreuzschraffur ist der Bereich der Dichtmembran 41 dargestellt, in dem diese eine konstante Dicke bzw. Wandstärke aufweist. Die die Dichtlippen 43, 44 bildenden Bereiche der Dichtmembran 41 sind in linearer Schraffur von links unten nach rechts oben bzw. von links oben nach rechts unten dargestellt.

Bei dem in Figur 7 dargestellten Beispiel sind die die Dichtlippen 43, 44 bildenden
Bereiche nicht wie bei den oben anhand der Figuren 3 bis 6 dargestellten Dichtmembranen rechteckig. Stattdessen verlaufen die Grenzen zwischen den die Dichtlippen 43, 44 bildenden Bereichen und dem diese umgebenden Bereich, in dem die Dichtmembran 41 eine konstante Dicke aufweist, bogenförmig. Die die Dichtlippen 43, 44 bildenden Bereiche sind insbesondere in der Mitte des Schlitzes 42 breiter und werden zu den Enden des Schlitzes 42 hin schmäler.

Die Keilform der die Dichtlippen 43, 44 bildenden Bereiche kann entlang des Schlitzes 42 (d.h. in Figur 7 von links nach rechts) konstant sein, so dass die Dicke der Dichtmembran 41 entlang des gesamten Schlitzes 42 konstant und überall die gleiche Funktion des Abstands vom Schlitz 42 ist. Es resultiert eine Stufe mit als Funktion des Ortes variierender Höhe zwischen dem eine Dichtlippe 43, 44 bildenden Bereich und dem umgebenden Bereich der Dichtmembran 41 mit konstanter Dicke.

Alternativ ist der Öffnungswinkel der Keilform der die Dichtlippen 43, 44 bildenden Bereiche überall gleich, und die Dicke der Dichtlippen 43, 44 unmittelbar am Spalt 42 variiert entlang des Spalts 42 derart, dass die die Dichtlippen 43, 44 bildenden Bereiche stufenlos in den umgebenden Bereich der Dichtmembran 41 mit konstanter Dicke übergehen. Gemäß einer weiteren Alternative variiert die Keilform (insbesondere der Öffnungswinkel) der die Dichtlippen 43, 44 bildenden Bereiche entlang des Schlitzes 42 derart, dass die Dicke der Dichtlippen 43, 44 entlang des Schlitzes 42 konstant ist und die die Dichtlippen 43, 44 bildenden Bereiche stufenlos in den umgebenden Bereich der Dichtmembran 41 mit konstanter Dicke übergehen.

Die beschriebenen Beispiele zeigen, dass die elastischen Eigenschaften der Dichtlippen 43, 44 in weiten Grenzen variieren bzw. eingestellt werden können, um ein optimales Anliegen der Dichtlippen 43, 44 an medizinischen Instrumenten mit einem breiten Spektrum von Querschnitten und insbesondere Durchmessern zu ermöglichen. Dabei können beide aneinander anliegenden Dichtmembranen gleich oder alternativ unterschiedlich ausgestaltete Dichtlippen aufweisen.

Die anhand der Figuren 3 bis 7 dargestellten Beispiele von Dichtmembranen 41,
51 sind im Wesentlichen plattenförmig und eben.

Beschreibung der Ausführungsformen der Erfindung

Nachfolgend werden anhand der Figuren
8 bis 13 Ausführungsformen gekrümmter, kuppelförmig gewölbter Dichtmembranen dargestellt. Für die Dichtlippen gilt dabei das anhand der Figuren 3 bis 7 Beschriebene entsprechend.

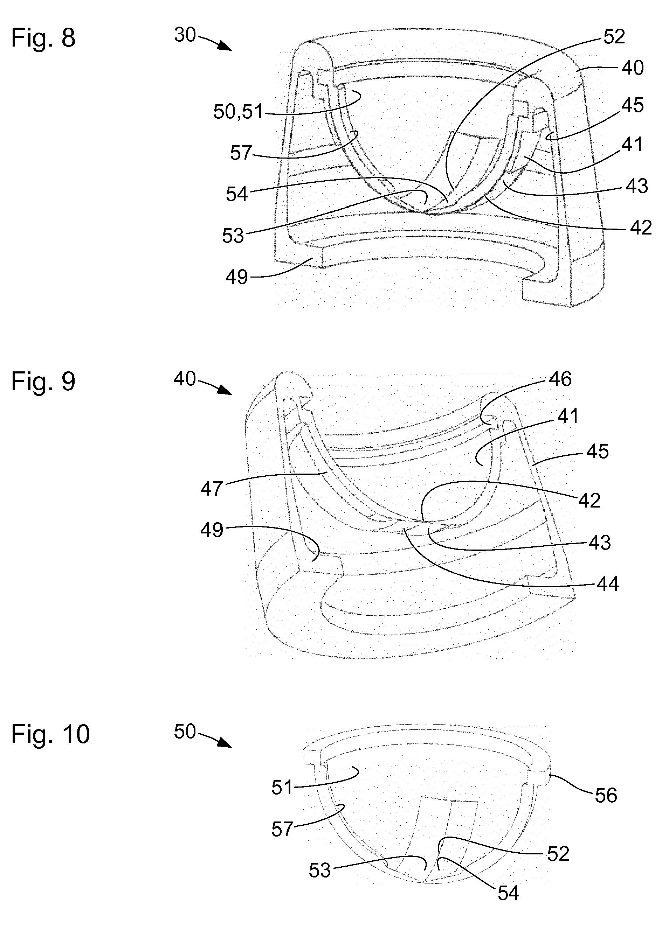

Figur 8 zeigt eine schematische axonometrische Darstellung eines Ausführungsbeispiels einer Dichteinrichtung 30. Die Dichteinrichtung 30 ist in Figur 8 entlang einer Ebene aufgeschnitten dargestellt, die die Längsachse 28 (vgl. Figur 1) eines Trokars 20 bzw. eines Tubus eines Trokars, für den die Dichteinrichtung 30 vorgesehen ist, enthält.

Die Dichteinrichtung 30 umfasst ein erstes, einstückig ausgebildetes Dichtmembranbauteil 40 und ein zweites, einstückig ausgebildetes Dichtmembranbauteil 50. Das erster Dichtmembranbauteil 40 und das zweite Dichtmembranbauteil 50 weisen jeweils Silikon oder ein anderes elastisches Material auf. Beide Dichtmembranbauteile 40, 50 können das gleiche oder unterschiedliche Materialien aufweisen.

Das erste Dichtmembranbauteil 40 umfasst eine erste Dichtmembran 41 mit einem ersten Schlitz 42 in der ersten Dichtmembran 41. Der erste Schlitz 42 in der ersten Dichtmembran 41 liegt in der Schnittebene. Deshalb ist nur eine der beiden an den ersten Schlitz 42 angrenzenden Dichtlippen 43 in Figur 8 sichtbar.

Das erste Dichtmembranbauteil 40 umfasst ferner einen elastischen mantelförmigen Wandabschnitt 45. Der elastische mantelförmige Wandabschnitt 45 weist im Wesentlichen die Gestalt eines Ausschnitts eines Kegelmantels auf und liegt ringförmig um die erste Dichtmembran 41 und das zweite Dichtmembranbauteil 50. Der elastische Wandabschnitt 45 verbindet den äußeren Rand der ersten Dichtmembran 41 mit einem Randprofil 49. Das Randprofil 49 bildet einen Befestigungsbereich zur formschlüssigen Verbindung der Dichteinrichtung 30 mit einem proximalen Ende eines Trokars 20 (vgl. Figuren 1 und 2) bzw. eines Tubus eines Trokars.

Das zweite Dichtmembranbauteil 50 umfasst eine zweite Dichtmembran 51 mit einem zweiten Schlitz 52 zwischen Dichtlippen 53, 54. Der zweite Schlitz 52 ist senkrecht zum ersten Schlitz 42 in der ersten Dichtmembran 41 und senkrecht zu der Schnittebene der Figur 8 angeordnet. Ferner umfasst das zweite Dichtmembranbauteil 50 beiderseits des zweiten Schlitzes 52 je eine Verstärkungsrippe 57. Die Verstärkungsrippen 57 sind im Wesentlichen senkrecht und mittig zum zweiten Schlitz 52 angeordnet und erstrecken sich jeweils vom äußeren Rand der zweiten Dichtmembran 51 bis in die Nähe der Dichtlippen 53, 54. Jede Verstärkungsrippe 57 hat beispielsweise einen halbkreisförmigen, halbelliptischen oder rechteckigen Querschnitt.

Die Elastizität des elastischen mantelförmigen Wandabschnitts 45 ermöglicht eine Bewegung der Dichtmembranen 41, 51 relativ zu den durch das Randprofil 49 gebildeten Befestigungsbereich der Dichteinrichtung 30, insbesondere eine Verschiebung senkrecht zur Längsachse 28 (vgl. Figur 1) und/oder ein Kippen oder Schwenken um Achsen senkrecht zur Längsachse 28.

Figur 9 zeigt eine weitere schematische axonometrische Darstellung des ersten Dichtmembranbauteils 40 des Ausführungsbeispiels aus Figur 8. Auch in Figur 9 ist das erste Dichtmembranbauteil 40 entlang einer Ebene aufgeschnitten dargestellt, die die Längsachse 28 (vgl. Figur 1) enthält. Die Schnittebene der Figur 9 ist jedoch senkrecht zur Schnittebene der Figur 8 und damit senkrecht zum ersten Schlitz 42 in der ersten Dichtmembran 41. Dadurch und durch die andere Blickrichtung sind in Figur 9 beide den ersten Schlitz 42 bildende Dichtlippen 43, 44 erkennbar.

Ferner ist ein Paar Verstärkungsrippen 47 an der vom zweiten Dichtmembranbauteil 50 abgewandten distalen Seite bzw. Oberfläche der ersten Dichtmembran 41 erkennbar. Die Verstärkungsrippen 47 sind symmetrisch, senkrecht und mittig zum ersten Schlitz 42 angeordnet. Auch die Verstärkungsrippen 47 am zweiten Dichtmembranbauteil 50 können jeweils einen halbkreisförmigen, halbelliptischen, rechteckigen oder anderen Querschnitt aufweisen und erstrecken sich vom äußeren Rand der ersten Dichtmembran 41 bis in die Nähe der Dichtlippen 43, 44.

Ferner ist in Figur 9 ein erstes Randprofil 46 am Rand der ersten Dichtmembran 41 erkennbar. Das erste Randprofil 46 wird durch eine umlaufende, zur Längsachse hin offene Nut gebildet.

Figur 10 zeigt eine weitere schematische axonometrische Darstellung des zweiten Dichtmembranbauteils 50 des Ausführungsbeispiels der Figuren 8 und 9. Auch in Figur 10 ist das zweite Dichtmembranbauteil 50 entlang einer Ebene aufgeschnitten gezeigt, die die Längsachse 28 (vgl. Figur 1) enthält. Die Schnittebene der Figur 10 entspricht der Schnittebene der Figur 8 und ist senkrecht zu der Schnittebene der Figur 9 und dem zweiten Schlitz 52 in der zweiten Dichtmembran 51.

In Figur 10 sind die bereits oben anhand der Figur 8 erläuterten Merkmale des zweiten Dichtmembranbauteils 50 erkennbar. Ferner ist ein zweites Randprofil 56 am äußeren Rand der zweiten Dichtmembran 51 bzw. des zweiten Dichtmembranbauteils 50 erkennbar. Das zweite Randprofil 56 weist die Gestalt eines nach radial außen ragenden Kragens auf. Das erste Randprofil 46 am ersten Dichtmembranbauteil 40 (vgl. Figur 9) und das zweite Randprofil 56 am zweiten Dichtmembranbauteil 50 weisen korrespondierende Querschnitte auf.

In Figur 8 ist erkennbar, dass das erste Randprofil 46 am ersten Dichtmembranbauteil 40 das zweite Randprofil 56 am zweiten Dichtmembranbauteil 50 aufnehmen kann. Damit bilden die Randprofile 46, 56 eine formschlüssige Verbindung zwischen den Dichtmembranbauteilen 40, 50, die aufgrund der Elastizität beider Dichtmembranbauteile 40, 50 wiederholt hergestellt und gelöst werden kann. Zum Lösen der formschlüssigen Verbindung, insbesondere zum Herausziehen des zweiten Randprofils 56 aus dem ersten Randprofil 46, kann am zweiten Dichtmembranbauteil 50 eine Lasche oder ein anderes Mittel vorgesehen sein, das beispielsweise mit zwei Fingern ergriffen werden kann, um das zweite Randprofil 56 aus dem ersten Randprofil 46 zu ziehen.

Das erste Dichtmembranbauteil 40 und das zweite Dichtmembranbauteil 50, insbesondere die erste Dichtmembran 41 und die zweite Dichtmembran 51 sowie die Randprofile 46, 56 sind so ausgebildet, dass die Dichtmembranen 41, 51 vollflächig aneinander anliegen, wenn das zweite Randprofil 56 im ersten Randprofil 46 aufgenommen ist. Um dabei die vorgesehene relative Ausrichtung der Schlitze 42, 52 in den Dichtmembranen 41, 51 sicherzustellen, sind die Randprofile 46, 56 insbesondere abweichend von den Darstellungen in den Figuren 8 bis 10 nicht vollständig kreissymmetrisch ausgebildet. Stattdessen ist beispielsweise durch eine Nase an einem der beiden Dichtmembranbauteile 40, 50 und eine korrespondierende Ausnehmung am anderen Dichtmembranbauteil 40, 50 eine vorbestimmte relative Orientierung festgelegt.

Bei dem in den Figuren 8 bis 10 dargestellten Ausführungsbeispiel sind die Dichtmembranen 41, 51 kuppelförmig gewölbt. Insbesondere sind die innere bzw. proximale Oberfläche der ersten Dichtmembran 41, die an der zweiten Dichtmembran 51 anliegt, und die äußere bzw. distale Oberfläche der zweiten Dichtmembran 51, die an der ersten Dichtmembran 41 anliegt, jeweils kugelflächenförmig ausgebildet. Alternativ kommen andere Formen in Betracht, beispielsweise die Gestalt eines zweiachsigen oder dreiachsigen Ellipsoids.

Die Oberflächen des ersten Dichtmembranbauteils 40 und des zweiten Dichtmembranbauteils 50 können teilweise oder vollständig reibungsmindernd beschichtet sein, beispielsweise mit Poly (p-Xylylene), das auch unter dem Markennamen Parylene vertrieben wird. Eine reibungsmindernde Beschichtung im Bereich der Dichtlippen 43, 44, 53, 54 kann eine Haft- und/oder Gleitreibung zwischen der Dichteinrichtung 30 und einem in die Dichteinrichtung 30 eingeführten medizinischen Instrument reduzieren. Eine reibungsmindernde Beschichtung an der inneren, proximalen und an der zweiten Dichtmembran 51 anliegenden Oberfläche der ersten Dichtmembran 41 sowie an der äußeren, distalen und an der ersten Dichtmembran 41 anliegenden Oberfläche der zweiten Dichtmembran 51 kann insbesondere die Haftreibung zwischen den Dichtmembranen 41, 51 reduzieren und damit Verschleiß verringern und die Dichtfunktion fördern.

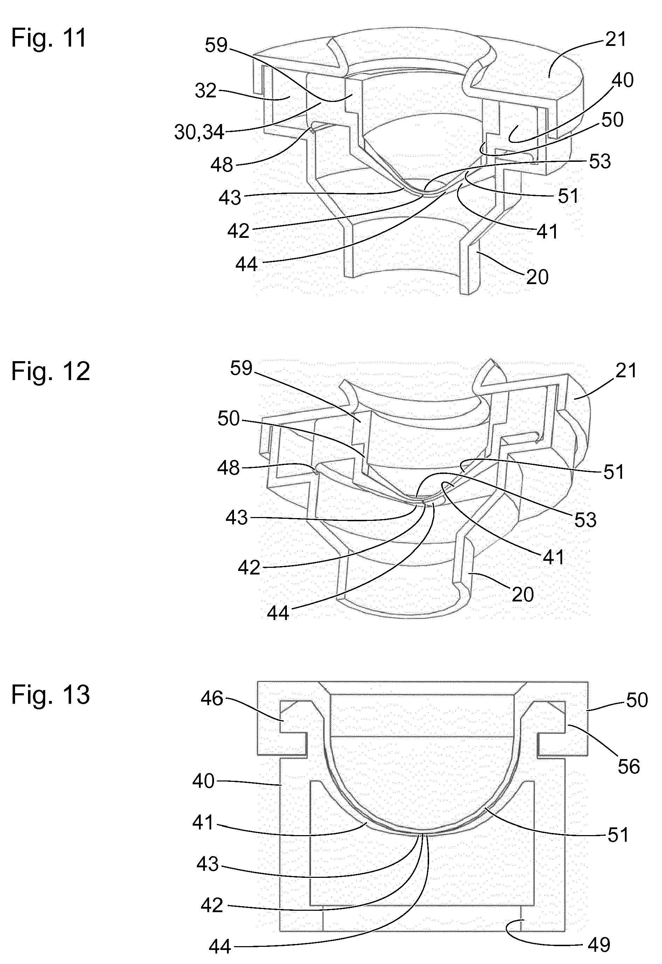

Die Figuren 11 und 12 zeigen schematische axonometrische Darstellungen eines weiteren Ausführungsbeispiels einer Dichteinrichtung 30. Ähnlich wie in den Figuren 8 bis 10 ist die Dichteinrichtung 30 in den Figuren 11 und 12 entlang einer Ebene aufgeschnitten dargestellt, die die Längsachse 28 (vgl. Figur 1) enthält. Die Schnittebenen der Figuren 11 und 12 sind gleich. Im Gegensatz zu den Darstellungen der Figuren 8 bis 10 ist nicht nur die Dichteinrichtung 30, sondern auch das proximale Ende eines Trokars 20 bzw. eines Tubus eines Trokars dargestellt. Die Darstellungen der Figuren 11 und 12 unterscheiden sich nur in den Blickrichtungen, aus denen das Ausführungsbeispiel dargestellt ist. Die nachfolgenden Ausführungen beziehen sich deshalb immer gleichzeitig auf die Figuren 11 und 12.

Das proximale Ende des Trokars 20 weist eine stufenförmige Erweiterung seines Querschnitts auf, die zusammen mit einem Deckel 21 einen Hohlraum 32 zur Aufnahme der Dichteinrichtung 30 bzw. eines scheibenförmigen Rands 34 der Dichteinrichtung 30 bildet. Der scheibenförmige Rand 34 und der Hohlraum 32 sind so aufeinander abgestimmt, dass die Dichteinrichtung 30 in Richtung parallel zur Längsachse 28 (vgl. Figur 1) spielarm oder spielfrei geführt ist und in den Richtungen senkrecht zur Längsachse 28 einen vorbestimmten Spielraum hat, innerhalb dessen die Dichteinrichtung 30 verschoben werden kann. Anders ausgedrückt ist die Dichteinrichtung 30 in dem Hohlraum 32 zwischen der stufenförmigen Erweiterung des Trokars 20 und dem Deckel 21 in einer Ebene schwimmend gelagert. Am scheibenförmigen Rand 34 der Dichteinrichtung 30 ist eine ringförmige Gleitdichtung 48 vorgesehen, die an einer korrespondierenden ringförmigen Fläche am proximalen Ende des Trokars fluiddicht anliegt.

Die Dichteinrichtung 30 umfasst ähnlich wie das oben anhand der Figuren 8 bis 10 dargestellte Ausführungsbeispiel ein erstes Dichtmembranbauteil 40 und ein zweites Dichtmembranbauteil 50, die jeweils Silikon oder ein anderes elastisches Material aufweisen. Das erste Dichtmembranbauteil 40 umfasst den scheibenförmigen Rand 34 der Dichteinrichtung 30 und eine erste Dichtmembran 41 mit einem ersten Schlitz 42 senkrecht zu den Schnittebenen der Figuren 11 und 12 zwischen Dichtlippen 43, 44. Das zweite Dichtmembranbauteil 50 umfasst eine zweite Dichtmembran 51 mit einem zweiten Schlitz 52 in der Schnittebene der Figuren 11 und 12 und zwischen einer Dichtlippe 53 und einer weiteren Dichtlippe, die in den Figuren 11 und 12 nicht dargestellt ist.

Das zweite Dichtmembranbauteil 50 wird durch Formschluss mit dem ersten Dichtmembranbauteil 40 oder durch die Wirkung des Deckels 21 in der in den Figuren 11 und 12 dargestellten vorgesehenen Position relativ zum ersten Dichtmembranbauteil 40 gehalten. In dieser relativen Position der Dichtmembranbauteile 40, 50 liegen die Dichtmembranen 41, 51 vollflächig aneinander an, ähnlich wie oben anhand der Figuren 8 bis 10 beschrieben.

Insbesondere in Figur 11 ist eine Nase 59 am zweiten Dichtmembranbauteil 50 erkennbar. Die Nase 59 greift in eine entsprechende Ausnehmung am ersten Dichtmembranbauteil 40 ein und erzwingt durch Formschluss eine vorbestimmte relative Orientierung der Dichtmembranbauteile 40, 50 und damit auch der Schlitze 42, 52.

Die Dichtmembranen 41, 51 sind im weiteren Sinne kuppelförmig, jedoch nicht vollständig kugelflächenförmig gewölbt. Lediglich in einem mittleren Bereich nahe dem Kreuzungspunkt der beiden Schlitze 42, 52 sind die Dichtmembranen 41, 51 kugelflächenförmig oder im Wesentlichen kugelflächenförmig gewölbt. An diesen mittleren Bereich schließt sich ein ringförmiger Bereich an, in dem die Dichtmembranen 41, 51 jeweils im Wesentlichen die Gestalt eines Ausschnitts eines Kegelmantels aufweisen. Die Dichtmembranen 41,51 weisen jeweils zu ihren äußeren Rändern hin zunehmende Dicken bzw. Wandstärken auf.

Die Oberflächen der Dichtmembranbauteile 40, 50 können teilweise oder vollständig reibungsmindernd beschichtet sein.

Eine Bewegbarkeit, insbesondere eine Verschiebbarkeit der Dichtmembranen 41, 51 relativ zu einem Befestigungsbereich bzw. zu einem Trokar wird beim Ausführungsbeispiel der Figuren 8 bis 10 durch die Elastizität des elastischen mantelförmigen Wandabschnitts 45 und beim Ausführungsbeispiel der Figuren 11 und 12 durch eine Verschiebbarkeit des scheibenförmigen Rands 34 der Dichteinrichtung 30 im korrespondierenden Hohlraum 32 bewirkt. Eine Führung eines in die Dichteinrichtung einzuführenden medizinischen Instruments zur Mitte der Dichtmembranen 41, 51 hin wird bei dem Ausführungsbeispiel der Figuren 8 bis 10 durch eine kuppelförmige Wölbung oder insbesondere eine näherungsweise kugelförmige Ausgestaltung und beim Ausführungsbeispiel der Figuren 11 und 12 durch eine näherungsweise konische Ausgestaltung der Dichtmembranen 41, 51 bewirkt. Die kuppelförmige Wölbung oder insbesondere näherungsweise kugelflächenförmige Ausgestaltung der Dichtmembranen 41, 51 des Ausführungsbeispiels der Figuren 8 bis 10 ist auch bei einer Verschiebbarkeit der Dichteinrichtung 30, wie sie beim Ausführungsbeispiel der Figuren 11 und 12 vorliegt, möglich. Umgekehrt ist auch bei einer Verschiebbarkeit der Dichtmembranen 41, 51 relativ zum Befestigungsbereich 49 aufgrund der Elastizität eines mantelförmigen Wandabschnitts 45, wie sie beim Ausführungsbeispiel der Figuren 8 bis 10 vorliegt, eine Ausgestaltung der Dichtmembranen 41, 51, wie sie beim Ausführungsbeispiel der Figuren 11 und 12 vorliegt, möglich.

Figur 13 zeigt eine schematische Schnittansicht eines weiteren Ausführungsbeispiels einer Dichteinrichtung 30. Ähnlich wie das Ausführungsbeispiel der Figuren 8 bis 10 umfasst die Dichteinrichtung 30 ein erstes Dichtmembranbauteil 40 mit einer ersten Dichtmembran 41 und einem einen Verbindungsbereich bildenden Randprofil 49 sowie ein zweites Dichtmembranbauteil 50 mit einer zweiten Dichtmembran 51. Die Dichteinrichtung 30 unterscheidet sich von dem Ausführungsbeispiel der Figuren 8 bis 10 unter anderem in der Ausgestaltung von Randprofilen 46, 56 an den Rändern der Dichtmembranen 41, 51 zur formschlüssigen Verbindung der Dichtmembranbauteile 40, 50.

Ferner unterscheidet sich die Dichteinrichtung 30 von dem Ausführungsbeispiel der Figuren 8 bis 10 dadurch, dass die Dicken bzw. Wandstärken der Dichtmembranen 41,51 von den Schlitzen 42, 52 zum äußeren Rand jeweils kontinuierlich zunehmen. Der erste Schlitz 42 in der ersten Dichtmembran 41 liegt senkrecht zur Schnittebene der Figur 13, die Zunahme der Dicke der ersten Dichtmembran 41 vom ersten Schlitz 42 zum Rand der ersten Dichtmembran 41 ist erkennbar. Der zweite Schlitz 52 in der zweiten Dichtmembran 51 liegt in der Schnittebene der Figur 13. Die in Figur 13 sichtbare Dicke bzw. Wandstärke der zweiten Dichtmembran 51 unmittelbar am zweiten Schlitz 52 ist deshalb im Wesentlichen konstant.

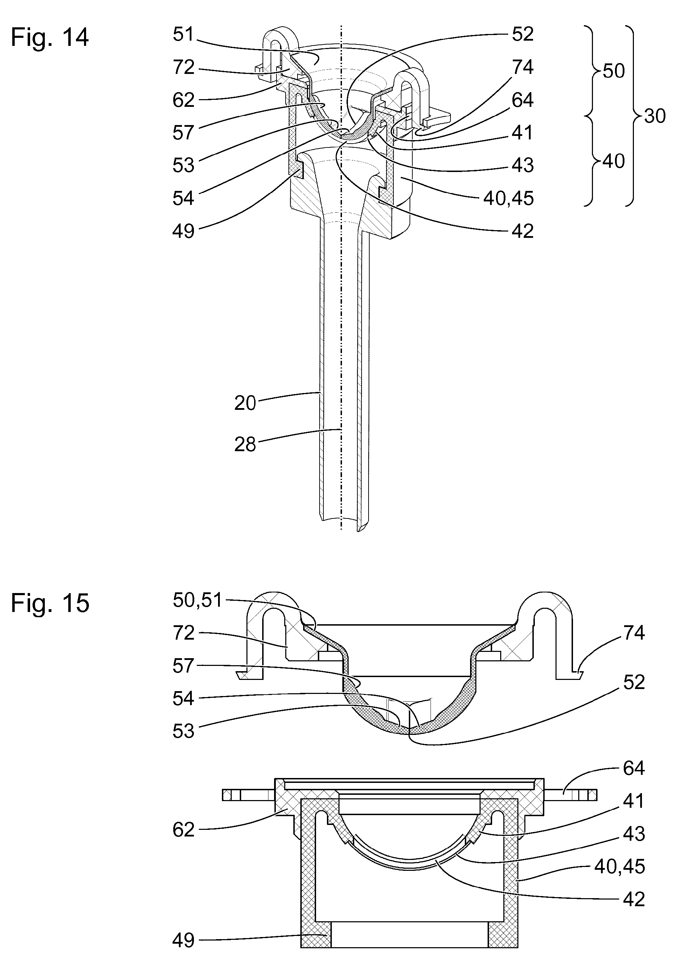

Figur 14 zeigt eine schematische axonometrische Schnittdarstellung eines weiteren Trokars 20 mit einer weiteren Dichteinrichtung 30. Die Schnittebene enthält die Längsachse 28 des Trokars 20 und entspricht insbesondere den Schnittebenen der Figuren 1, 2, 4, 8, 10, 11, 12,13.

Der Trokar 20 ähnelt weitgehend dem oben anhand der Figuren 1 und 2 dargestellten Trokar. Die Dichteinrichtung 30 ähnelt in vielen Merkmalen, Eigenschaften und Funktionen der oben anhand der Figuren 8 bis 10 dargestellten Dichteinrichtung, insbesondere hinsichtlich der kuppelförmigen Wölbung der Dichtmembranen 41, 51, der Anordnung der Schlitze 42, 52 und der Gestalt der Dichtlippen 43, 44, 53, 54.

Die Dichtmembranen 41, 51 können jeweils in einem äußeren ringförmigen Bereich kegelmantelförmig und nur in einem zentralen Bereich kuppelförmig, insbesondere kugelflächenförmig gewölbt sein und insoweit der oben anhand der Figuren 11 und 12 dargestellten Dichteinrichtung ähneln. Ferner können die Dichtmembranen 41,51 abweichend von der Darstellung in Figur 14 jeweils eine Gestalt aufweisen, wie sie oben anhand der Figur 13 dargestellt ist.

Nachfolgend sind nur jene Merkmale und Eigenschaften der Dichteinrichtung 30 dargestellt, in denen sich die Dichteinrichtung 30 von den Ausführungsformen der Figuren 8 bis 13, insbesondere von der Ausführungsform der Figuren 8 bis 10 unterscheidet.

Das erste Dichtmembranbauteil 40 umfasst insbesondere die erste Dichtmembran 41 und einen ersten Rahmen 62. Das zweite Dichtmembranbauteil 50 umfasst insbesondere die zweite Dichtmembran 51 und einen zweiten Rahmen 72. Der erste Rahmen 62 und der zweite Rahmen 72 weisen jeweils ein Material auf, das deutlich steifer oder weniger elastisch ist als das Material oder die Materialien der Dichtmembranen 41, 51. Die erste Dichtmembran 41 ist mit dem ersten Rahmen 62 gefügt, insbesondere an einer ringförmigen Fläche geklebt, geschweißt oder auf andere Weise stoffschlüssig gefügt. Alternativ können die erste Dichtmembran 41 und der erste Rahmen 62 während eines Spritzgussvorgangs oder eines anderen Gussvorgangs miteinander stoffschlüssig gefügt werden. Die zweite Dichtmembran 51 ist mit dem zweiten Rahmen 72 gefügt, insbesondere an einer ringförmigen Fläche geklebt, geschweißt oder auf andere Weise stoffschlüssig gefügt. Alternativ können die zweite Dichtmembran 51 und der zweite Rahmen 72 während eines Spritzgussvorgangs oder eines anderen Gussvorgangs miteinander stoffschlüssig gefügt sein.

Alternativ und abweichend von der Darstellung in Figur 14 sind die erste Dichtmembran 41 und der erste Rahmen 62 aus dem gleichen Material und insbesondere gleichzeitig und von Anfang an einstückig hergestellt. Auch die zweite Dichtmembran 51 und der zweite Rahmen 72 können aus dem gleichen Material und insbesondere gleichzeitig und von Anfang an einstückig hergestellt sein. In diesen Fällen weisen der erste Rahmen 62 und der zweite Rahmen 72 aufgrund deutlich größerer Materialstärken eine deutlich geringere Elastizität auf als die erste Dichtmembran 41 und die zweite Dichtmembran 51.

Am ersten Rahmen 62 sind einander gegenüberliegend zwei Ausnehmungen, insbesondere Ösen 64 vorgesehen. Am zweiten Rahmen 72 sind einander gegenüberliegend zwei Rastnasen 74 vorgesehen. U-förmige Abschnitte zwischen dem Rahmen 72 und den Rastnasen 74 wirken als elastische Elemente und ermöglichen Bewegungen der Rastnasen 74 gegen elastische Kräfte. Die Ösen 64 am ersten Rahmen 62 sind korrespondierend zu den Rastnasen 74 am zweiten Rahmen 72 angeordnet und ausgebildet. Bei der vorgesehenen und in Figur 14 dargestellten relativen Anordnung der Dichtmembranbauteile 40, 50 greift jeweils eine Rastnase 74 in eine Öse 64 ein und bildet mit dieser eine Rastverbindung. Die Rastverbindungen zwischen den Rastnasen 74 und den Ösen 64 halten die Dichtmembranbauteile 40, 50 in ihrer vorgesehenen und in Figur 14 dargestellten relativen Anordnung.

Figur 15 zeigt eine weitere schematische Schnittdarstellung der Dichteinrichtung 30 aus Figur 14. Die Schnittebene der Figur 15 enthält die Längsachse 28 (vg. Figur 14) und entspricht der Schnittebene der Figur 14. In Figur 15 sind die Dichtmembranbauteile 40, 50 voneinander mechanisch getrennt und beabstandet dargestellt. Das erste Dichtmembranbauteil 40 ist im unteren Teil, das zweite Dichtmembranbauteil 50 ist im oberen Teil der Figur 15 dargestellt.

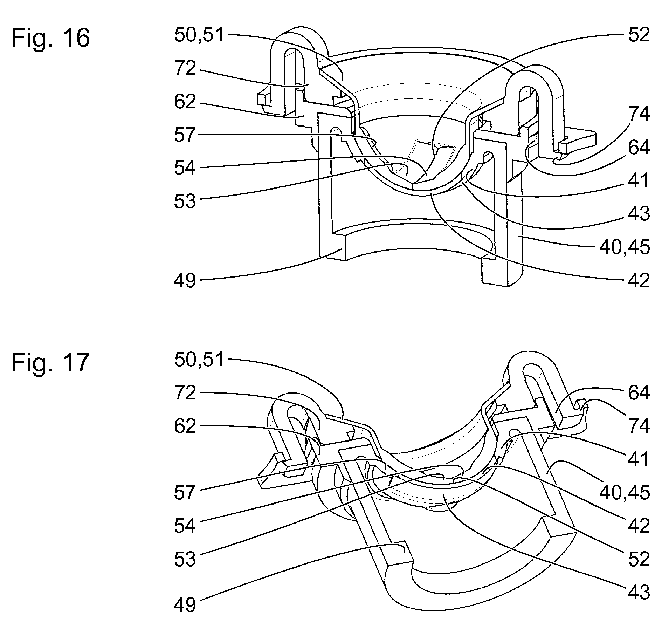

Die Figuren 16 und 17 zeigen weitere schematische axonometrische Schnittdarstellungen der Dichteinrichtungen 30 aus den Figuren 14 und 15. Die Schnittebenen der Figuren 16 und 17 enthalten die Längsachse 28 (vgl. Figur 14) und entsprechen den Schnittebenen der

Figuren 14 und 15. Ähnlich wie bei der Darstellung in Figur 14 sind die Dichtmembranbauteile 40, 50 in ihrer vorgesehenen relativen Anordnung und mechanisch miteinander verbunden dargestellt.

Ausgehend von der in den Figuren 14, 16 und 17 dargestellten Konfiguration bzw. Situation können durch manuellen Ausübung einer Kraft auf die Rastnasen 74 die Rastverbindungen gelöst werden. Danach können die Dichtmembranbauteile 40, 50 voneinander getrennt und dann entsorgt oder gereinigt und sterilisiert werden. Nach der Reinigung oder nach der Sterilisierung können die Dichtmembranbauteile 40, 50 durch Herstellen der Rastverbindungen wieder miteinander mechanisch verbunden werden.

Abweichend von den Darstellungen anhand der Figuren 14 bis 17 kann eine andere Zahl von Rastnasen 74 und Ösen 64 und eine entsprechende andere Zahl von Rastverbindungen zwischen den Dichtmembranbauteilen 40, 50 vorgesehen sein. Insbesondere sind jeweils drei oder jeweils vier Ösen 64, Rastnasen 74 und Rastverbindungen vorgesehen.

### Bezugszeichen

- 20: Trokar
- 21: Deckel am Trokar 20
- 22: medizinisches Instrument
- 24: Schaft des medizinischen Instruments 22
- 26: Bauchdecke eines Patienten
- 27: Hohlraum unter der Bauchdecke 26
- 28: Längsachse des Trokars 20
- 29: Richtung des Einführens eines medizinischen Instruments 22 in den Trokar 20
- 30: Dichteinrichtung
- 32: Hohlraum zur Aufnahme der Dichteinrichtung 30
- 34: scheibenförmiger Rand der Dichteinrichtung 30
- 40: erstes Dichtmembranbauteil
- 41: erste Dichtmembran am ersten Dichtmembranbauteil 40
- 42: erster Schlitz in der ersten Dichtmembran 41
- 43: erste Dichtlippe der ersten Dichtmembran 41
- 44: zweite Dichtlippe der ersten Dichtmembran 41
- 45: elastischer Wandabschnitt am ersten Dichtmembranbauteil 40
- 46: erstes Randprofil am Rand der ersten Dichtmembran 41
- 47: Verstärkungsrippe an der ersten Dichtmembran 41
- 48: Gleitdichtung am ersten Dichtmembranbauteil 40
- 49: Randprofil am ersten Dichtmembranbauteil 40
- 50: zweites Dichtmembranbauteil
- 51: zweite Dichtmembran am zweiten Dichtmembranbauteil 50
- 52: zweiter Schlitz in der zweiten Dichtmembran 51
- 53: erste Dichtlippe der zweiten Dichtmembran 51
- 54: zweite Dichtlippe der zweiten Dichtmembran 51
- 56: zweites Randprofil am Rand der zweiten Dichtmembran 51
- 57: Verstärkungsrippe an der zweiten Dichtmembran 51
- 59: Nase am zweiten Dichtmembranbauteil 50
- 62: erster Rahmen am ersten Dichtmembranbauteil 40
- 64: Rastnase am ersten Rahmen 62
- 72: zweiter Rahmen am zweiten Dichtmembranbauteil 50
- 74: Ausnehmung am zweiten Rahmen 72

## Patentansprüche

1. **Dichteinrichtung** (30) zur Abdichtung einer Durchführung für ein medizinisches Instrument (22), mit:
einer **ersten Dichtmembran** (41) aus einem elastischen Material mit einem ersten Schlitz (42);
einer **zweiten Dichtmembran** (51) aus einem elastischen Material mit einem zweiten Schlitz (52),
wobei die erste Dichtmembran (41) und die zweite Dichtmembran (51) flächig **aneinander anliegen,**
wobei die erste Dichtmembran (41) und die zweite Dichtmembran (51) so angeordnet sind, dass der erste Schlitz (42) und der zweite Schlitz (52) **nicht parallel** zu einander sind,
wobei der erste Schlitz (42) und der zweite Schlitz (52) jeweils **unverzweigt** sind,
**dadurch gekennzeichnet, dass** die erste Dichtmembran (41) und die zweite Dichtmembran (51) jeweils zumindest in einem mittigen Bereich **kuppelförmig gewölbt** sind.

2. Dichteinrichtung (30) nach dem vorangehenden Anspruch, bei der zumindest entweder die **Dicke** der ersten Dichtmembran (41) zum ersten Schlitz (42) hin oder die Dicke der zweiten Dichtmembran (51) zum zweiten Schlitz (52) hin kontinuierlich oder diskontinuierlich **abnimmt.**

3. Dichteinrichtung (30) nach einem der vorangehenden Ansprüche, bei der zumindest entweder die Dicke der ersten Dichtmembran (41) in beiderseits an den ersten Schlitz (42) angrenzenden Bereichen **keilförmig** zum ersten Schlitz (42) hin abnimmt oder die Dicke der zweiten Dichtmembran (51) in beiderseits an den zweiten Schlitz (52) angrenzenden Bereichen keilförmig zum zweiten Schlitz (52) hin abnimmt.

4. Dichteinrichtung (30) nach einem der vorangehenden Ansprüche, bei der die Länge des ersten Schlitzes (42) und die **Länge** des zweiten Schlitzes (52) jeweils mindestens **doppelt** so groß sind wie der größte Durchmesser eines Abschnitts (24) eines medizinischen Instruments (22), für den die Dichteinrichtung (30) vorgesehen ist.

5. Dichteinrichtung (30) nach einem der vorangehenden Ansprüche, bei der die erste Dichtmembran (41) und die zweite Dichtmembran (51) jeweils so ausgebildet sind, dass **jede** an einen Schlitz (42, 52) angrenzende **Dichtlippe** (43, 44, 53, 54) am größten kreisförmigen Querschnitt eines medizinischen Instruments (22), für den die Dichteinrichtung (30) vorgesehen ist, jeweils in einem Bereich anliegt, der sich in umfänglicher Richtung über **mindestens 90 Grad** erstreckt.

6. Dichteinrichtung (30) nach einem der vorangehenden Ansprüche, bei der die erste Dichtmembran (41) und die zweite Dichtmembran (51) mittels einer oder mehrerer Rastverbindungen (64, 74) miteinander mechanisch verbunden sind.

7. Dichteinrichtung (30) nach einem der vorangehenden Ansprüche, bei der der erste Schlitz (42) und der zweite Schlitz (52) jeweils **gerade** oder **bogenförmig** mit einem Krümmungsradius, der mindestens so groß ist wie eine Länge, eine Breite oder ein Durchmesser der ersten Dichtmembran (41) oder der zweiten Dichtmembran (51), ist.

8. Dichteinrichtung (30) nach einem der vorangehenden Ansprüche, bei der die erste Dichtmembran (41) und die zweite Dichtmembran (51) jeweils einen an die Schlitze (42, 52) angrenzenden **kugelflächenförmigen** Oberflächenabschnitt aufweisen.

9. Dichteinrichtung (30) nach einem der vorangehenden Ansprüche, bei der am äußeren Rand der ersten Dichtmembran (41) ein erstes **Randprofil** (46) und am äußeren Rand der zweiten Dichtmembran (51) ein zweites **Randprofil** (56) vorgesehen sind, wobei das erste Randprofil (46) und das zweite Randprofil (56) für eine **formschlüssige** Verbindung der ersten Dichtmembran (41) und der zweiten Dichtmembran (51) ausgebildet sind.

10. Dichteinrichtung (30) nach einem der vorangehenden Ansprüche, bei der sowohl der äußere Rand der ersten Dichtmembran (41) als auch der äußere Rand der zweiten Dichtmembran (51) jeweils an zumindest einer Stelle **von einer Kreissymmetrie so abweichen,** dass eine vorbestimmte relative Orientierung der Dichtmembranen (41, 51) vorgegeben ist.

11. Dichteinrichtung (30) nach einem der vorangehenden Ansprüche, ferner mit zumindest entweder einer **Verstärkungsrippe** (47) an der ersten Dichtmembran (41) oder einer Verstärkungsrippe (57) an der zweiten Dichtmembran (51).

12. Dichteinrichtung (30) nach einem der vorangehenden Ansprüche, bei der zumindest entweder die erste Dichtmembran (41) oder die zweite Dichtmembran (51) zumindest an einer Seite **reibungsmindernd beschichtet** ist.

13. Dichteinrichtung (30) nach einem der vorangehenden Ansprüche, ferner mit:
einem **Befestigungsbereich** (49) zur Befestigung der Dichteinrichtung (30) an einem Trokar (20) oder einer anderen medizinischen Vorrichtung;
einem die zweite Dichtmembran (51) umgebenden elastischen **mantelförmigen**
**Wandabschnitt** (45), der die zweite Dichtmembran (51) mit dem Befestigungsbereich (49) verbindet, und dessen Elastizität eine Bewegung der Dichtmembranen (41, 51) relativ zum Befestigungsbereich (49) ermöglicht.

14. **Trokar** (20) mit einer Dichteinrichtung (30) nach einem der vorangehenden Ansprüche.

## Claims

1. Sealing device (30) for sealing a feedthrough for a medical instrument (22), with:
a first sealing membrane (41) made of an elastic material and having a first slit (42);
a second sealing membrane (51) made of an elastic material and having a second slit (52),
wherein the first sealing membrane (41) and the second sealing membrane (51) lie flat on each other,
wherein the first sealing membrane (41) and the second sealing membrane (51) are arranged such that the first slit (42) and the second slit (52) are not parallel to each other,
wherein the first slit (42) and the second slit (52) are each unbranched,
**characterized in that** the first sealing membrane (41) and the second sealing membrane (51) are each bulged in a dome shape at least in a central area.

2. Sealing device (30) according to the preceding claim, in which at least either the thickness of the first sealing membrane (41) continuously or discontinuously decreases towards the first slit (42) or the thickness of the second sealing membrane (51) continuously or discontinuously decreases towards the second slit (52).

3. Sealing device (30) according to one of the preceding claims, in which at least either the thickness of the first sealing membrane (41) decreases in a wedge shape towards the first slit (42), in areas adjoining both sides of the first slit (42), or the thickness of the second sealing membrane (51) decreases in a wedge shape towards the second slit (52), in areas adjoining both sides of the second slit (52).

4. Sealing device (30) according to one of the preceding claims, in which the length of the first slit (42) and the length of the second slit (52) are each at least twice as great as the largest diameter of a portion (24) of a medical instrument (22) for which the sealing device (30) is provided.

5. Sealing device (30) according to one of the preceding claims, in which the first sealing membrane (41) and the second sealing membrane (51) are each designed such that each sealing lip (43, 44, 53, 54) adjacent to a slit (42, 52) bears on the largest circular cross section of a medical instrument (22) for which the sealing device (30) is provided, in each case in an area that extends over at least 90 degrees in the circumferential direction.

6. Sealing device (30) according to one of the preceding claims, in which the first sealing membrane (41) and the second sealing membrane (51) are mechanically connected to each other by means of one or more latching connections (64, 74).

7. Sealing device (30) according to one of the preceding claims, in which the first slit (42) and the second slit (52) are each straight or arcuate with a radius of curvature which is at least as great as a length, a width or a diameter of the first sealing membrane (41) or of the second sealing membrane (51).

8. Sealing device (30) according to one of the preceding claims, in which the first sealing membrane (41) and the second sealing membrane (51) each have a surface portion which is in the shape of a spherical surface and lies adjacent to the slits (42, 52).

9. Sealing device (30) according to one of the preceding claims, in which a first edge profile (46) is provided on the outer edge of the first sealing membrane (41), and a second edge profile (56) is provided on the outer edge of the second sealing membrane (51), wherein the first edge profile (46) and the second edge profile (56) are designed for a form-fit connection of the first sealing membrane (41) and the second sealing membrane (51).

10. Sealing device (30) according to one of the preceding claims, in which both the outer edge of the first sealing membrane (41) and the outer edge of the second sealing membrane (51) deviate, in each case at least at one point, from a circular symmetry, such that a predetermined relative orientation of the sealing membranes (41, 51) is predefined.

11. Sealing device (30) according to one of the preceding claims, moreover with at least either a reinforcement rib (47) on the first sealing membrane (41) or a reinforcement rib (57) on the second sealing membrane (51).

12. Sealing device (30) according to one of the preceding claims, in which at least either the first sealing membrane (41) or the second sealing membrane (51) has a friction-reducing coating at least on one side.

13. Sealing device (30) according to one of the preceding claims, moreover with:
a fastening area (49) for fastening the sealing device (30) on a trocar (20) or another medical device;
an elastic, jacket-shaped wall portion (45) which surrounds the second sealing membrane (51) and connects the second sealing membrane (51) to the fastening area (49), and of which the elasticity permits a movement of the sealing membranes (41, 51) relative to the attachment area (49).

14. Trocar (20) having a sealing device (30) according to one of the preceding claims.

## Revendications

1. Dispositif d'étanchéité (30) pour étanchéifier un passage pour un instrument médical (22), avec une première membrane d'étanchéité (41) en un matériau élastique avec une première fente (42);
une deuxième membrane d'étanchéité (51) en un matériau élastique avec une deuxième fente (52),
dans lequel la première membrane d'étanchéité (41) et la deuxième membrane d'étanchéité (51) sont appliquées à plat l'une sur l'autre,
dans lequel la première membrane d'étanchéité (41) et la deuxième membrane d'étanchéité (51) sont disposées de telle façon que la première fente (42) et la deuxième fente (52) ne soient pas parallèles l'une à l'autre,
dans lequel la première fente (42) et la deuxième fente (52) sont chacune non ramifiées,
**caractérisé en ce que** la première membrane d'étanchéité (41) et la deuxième membrane d'étanchéité (51) sont chacune bombées en forme de coupole au moins dans une région centrale.

2. Dispositif d'étanchéité (30) selon la revendication précédente, dans lequel au moins soit l'épaisseur de la première membrane d'étanchéité (41) diminue de façon continue ou discontinue en direction de la première fente (42) soit l'épaisseur de la deuxième membrane d'étanchéité (51) diminue de façon continue ou discontinue en direction de la deuxième fente (52).

3. Dispositif d'étanchéité (30) selon l'une quelconque des revendications précédentes, dans lequel au moins soit l'épaisseur de la première membrane d'étanchéité (41) diminue en forme de coin en direction de la première fente (42) dans des régions adjacentes de part et d'autre à la première fente (42) ou l'épaisseur de la deuxième membrane d'étanchéité (51) diminue en forme de coin en direction de la deuxième fente (52) dans des régions adjacentes de part et d'autre à la deuxième fente (52).

4. Dispositif d'étanchéité (30) selon l'une quelconque des revendications précédentes, dans lequel la longueur de la première fente (42) et la longueur de la deuxième fente (52) sont respectivement au moins deux fois plus grandes que le plus grand diamètre d'une partie (24) d'un instrument médical (22) pour laquelle le dispositif d'étanchéité (30) est prévu.

5. Dispositif d'étanchéité (30) selon l'une quelconque des revendications précédentes, dans lequel la première membrane d'étanchéité (41) et la deuxième membrane d'étanchéité (51) sont respectivement configurées de telle manière que chaque lèvre d'étanchéité (43, 44, 53, 54) adjacente à une fente (42, 52) s'applique respectivement sur la plus grande section transversale circulaire d'un instrument médical (22), pour laquelle le dispositif d'étanchéité (30) est prévu, dans une région qui s'étend sur au moins 90 degrés dans la direction périphérique.

6. Dispositif d'étanchéité (30) selon l'une quelconque des revendications précédentes, dans lequel la première membrane d'étanchéité (41) et la deuxième membrane d'étanchéité (51) sont mécaniquement reliées l'une à l'autre au moyen d'un ou de plusieurs assemblage(s) par encliquetage (64, 74).

7. Dispositif d'étanchéité (30) selon l'une quelconque des revendications précédentes, dans lequel la première fente (42) et la deuxième fente (52) sont respectivement droites ou courbes avec un rayon de courbure, qui est au moins aussi grand qu'une longueur, une largeur ou un diamètre de la première membrane d'étanchéité (41) ou de la deuxième membrane d'étanchéité (51).

8. Dispositif d'étanchéité (30) selon l'une quelconque des revendications précédentes, dans lequel la première membrane d'étanchéité (41) et la deuxième membrane d'étanchéité (51) présentent respectivement une partie de surface de forme sphérique adjacente à la fente (42, 52).

9. Dispositif d'étanchéité (30) selon l'une quelconque des revendications précédentes, dans lequel il est prévu sur le bord extérieur de la première membrane d'étanchéité (41) un premier profil de bord (46) et sur le bord extérieur de la deuxième membrane d'étanchéité (51) un deuxième profil de bord (56), dans lequel le premier profil de bord (46) et le deuxième profil de bord (56) sont configurés en vue d'un assemblage par emboîtement de la première membrane d'étanchéité (41) et de la deuxième membrane d'étanchéité (51).

10. Dispositif d'étanchéité (30) selon l'une quelconque des revendications précédentes, dans lequel aussi bien le bord extérieur de la première membrane d'étanchéité (41) que le bord extérieur de la deuxième membrane d'étanchéité (51) s'écartent en au moins un endroit d'une symétrie circulaire, de telle manière qu'il existe une orientation relative prédéterminée des membranes d'étanchéité (41, 51).

11. Dispositif d'étanchéité (30) selon l'une quelconque des revendications précédentes, avec en outre au moins soit une nervure de renforcement (47) sur la première membrane d'étanchéité (41) soit une nervure de renforcement (57) sur la deuxième membrane d'étanchéité (51).

12. Dispositif d'étanchéité (30) selon l'une quelconque des revendications précédentes, dans lequel au moins soit la première membrane d'étanchéité (41) soit la deuxième membrane d'étanchéité (51) est munie au moins sur un côté d'un revêtement réduisant le frottement.

13. Dispositif d'étanchéité (30) selon l'une quelconque des revendications précédentes, avec en outre:
une région de fixation (49) pour la fixation du dispositif d'étanchéité (30) à un trocart (20) ou à un autre dispositif médical;
une partie de paroi enveloppante élastique (45) entourant la deuxième membrane d'étanchéité (51), qui relie la deuxième membrane d'étanchéité (51) à la région de fixation (49), et dont l'élasticité permet un mouvement des membranes d'étanchéité (41, 51) par rapport à la région de fixation (49).

14. Trocart (20) avec un dispositif d'étanchéité (30) selon l'une quelconque des revendications précédentes.
